# EUROPEAN PATENT SPECIFICATION

(11) **EP 2 301 975 B1**
(45) Date of publication and mention of the grant of the patent: **24.07.2013**
(21) Application number: 09798001.5
(22) Date of filing: 17.07.2009
(51) Int. Cl.: C08F 2/50, A61K 6/083, C08F 20/28

(54) **POLYMERIZABLE COMPOSITION SUITABLE FOR LED LIGHT SOURCE**
POLYMERISIERBARE ZUSAMMENSETZUNG FÜR EINE LED-LICHTQUELLE
COMPOSITION POLYMÉRISABLE POUR UNE SOURCE DE LUMIÈRE LED

(30) Priority: 17.07.2008 JP 2008186005
(43) Date of publication of application: 30.03.2011
(73) Proprietor: KURARAY NORITAKE DENTAL INC., Kurashiki-shi Okayama 710-0801 (JP)
(72) Inventor: HINAMOTO, Ai, Kurashiki-shi Okayama 710-0801 (JP); ISHINO, Hiroshige, Frankfurt am Main 60488 (DE)
(74) Representative: Brunetti, Fabrizio
(86) International application number: PCT/JP2009/062992
(87) International publication number: WO 2010/008077

(56) References cited:
- JP-A- 2006 176 488
- JP-A- 2007 039 453
- JP-A- 2007 131 721
- JP-T- 2005 531 632
- ANTONUCCI, J. M. ET AL: "Effects of filler, initiator and cavity design factor on", POLYMER PREPRINTS (AMERICAN CHEMICAL SOCIETY, DIVISION OF POLYMER CHEMISTRY) , 49(2), 414-415 CODEN: ACPPAY; ISSN: 0032-3934, 2008, XP002684799,
- SKRTIC, D. ET AL: "Effect of chemical structure and composition of the resin phase on mechanical strength and vinyl conversion of amorphous calcium phosphate-based composites", JOURNAL OF BIOMEDICAL MATERIALS RESEARCH, PART A , 68A(4), 763-772 CODEN: JBMRCH, 2004, XP002684800,
- LISKA, ROBERT ET AL: "New photopolymers for rapid prototyping of cellular structures", POLYMER PREPRINTS (AMERICAN CHEMICAL SOCIETY, DIVISION OF POLYMER CHEMISTRY) , 45(2), 77-78 CODEN: ACPPAY; ISSN: 0032-3934, 2004, XP002684801,
- RICHARDS, N. D. ET AL: "Dental polymeric composites activated with", POLYMER PREPRINTS (AMERICAN CHEMICAL SOCIETY, DIVISION OF POLYMER CHEMISTRY) , 45(2), 362-363 CODEN: ACPPAY; ISSN: 0032-3934, 2004, XP002684802,
- NEUMANN, MIGUEL G. ET AL: "The initiating radical yields and the efficiency of different light curing units", DENTAL MATERIALS , 22(6), 576-584 CODEN: DEMAEP; ISSN: 0109-5641, 2006, XP002684803,

## Description

### TECHNICAL FIELD

The present invention relates to a polymerizable composition suitable for photopolymerization using a light emitting diode (LED) light source, particularly a polymerizable composition suitable for dental applications.

### BACKGROUND ART

In dental treatment, in order to repair dental caries and a lost part of a tooth, a photocurable dental material containing a photopolymerization initiator is filled into the dental caries and the lost part, and cured by being irradiated with light using a dental light-curing unit.

For example, Patent Literature 1 discloses, as a material used for tooth repair, a photocurable dental material containing a (meth)acrylate polymerizable monomer, an acylphosphine oxide polymerization initiator, and an amine compound.

Patent Literature 2 discloses that a polymerizable composition containing at least two different types of polymerization initiators, specifically a polymerizable composition containing camphorquinone and a bisacylphosphine oxide, is filled into dental caries.

Patent Literature 3 discloses a dental polymerizable composition containing, as its components, a monomer having a polymerizable olefinic unsaturated group, an acylphosphine oxide as a photopolymerization initiator, an organic peroxide as an initiator, and a tertiary amine as a reducing agent. The organic peroxide and the tertiary amine are packed separately.

Patent Literature 4 discloses a dental repairing material containing, as its components, a colloidal silica, a polymerizable monomer, and a polymerization initiator. It also discloses that α-diketone and a reducing agent, such as a tertiary amine, are used in combination as the polymerization initiator.

Conventionally, halogen lamp-curing units have been used as the dental light-curing units, but in recent years, LED light-curing units are replacing them because of their longer lamp lives and higher light utilization efficiencies (see Patent Literature 5, for example). However, although the light emitted from an LED light-curing unit has a peak wavelength similar to that of the light emitted from a halogen lamp-curing unit, its wavelength range is narrower and emission spectrum is different from that of the light emitted from a halogen lamp-curing unit. Accordingly, a photocurable dental materials shows different curing behaviors between the case where it is cured using an LED light-curing unit and the case where it is cured using a halogen lamp-curing unit. Thus, when a dental material as mentioned above is photocured using an LED light-curing unit, its curability is lowered and bond strength to a tooth structure is reduced in some cases.

On the other hand, Patent Literature 6 discloses a photopolymerizable composition that contains a radical polymerizable monomer and a photopolymerization initiator and exhibits excellent curability in both cases of using a halogen lamp-curing unit and an LED light-curing unit. The photopolymerization initiator contains a coumarin compound, a (bis)acylphosphine oxide, and a polymerization accelerator. Patent Literature 6 also discloses that amines can be used as the polymerization accelerator, and that the photopolymerization initiator further may contain α-diketones. However, as a result of studies made by the present inventors, it has been found that the composition is discolored during storage when it contains a quaternary-component photopolymerization initiator composed of a coumarin compound, a (bis)acylphosphine oxide, amines, and α-diketones. Discoloration of the compound in dental applications causes problems in terms of aesthetic appearance.

### CITATION LIST

### Patent Literature

PTL 1: JP 2000-16910A
PTL 2: EP 1468662
PTL 3: JP 10(1998)-338610A
PTL 4: JP 11(1999)-100305 A
PTL 5: JP 2000-271155 A
PTL 6: JP 2005-171213 A

### SUMMARY OF INVENTION

### Technical Problem

It is an object of the present invention to provide a polymerizable composition that exhibits high photocurability not only in the case of using a halogen lamp-curing unit but also in the case of using an LED light-curing unit, hardly has discoloration even when stored for a long time, and when applied to a dental material, a cured product of which has high bond strength to a tooth structure.

### Solution to Problem

The present invention that has achieved the above-mentioned object is a polymerizable composition including: a photopolymerization initiator composition containing a photopolymerization initiator component consisting of only a bisacylphosphine oxide (A) and α-diketone (B), and tertiary amine (C); and a polymerizable monomer (D). A weight ratio (A):(B) between the bisacylphosphine oxide (A) and the α-diketone (B) is 1:9 to 9:1. A weight ratio (D):(C) between the polymerizable monomer (D) and the tertiary amine (C) is 100:0.5 to 100:10.

The present invention also is a dental bonding material using the polymerizable composition.

### Advantageous Effects of Invention

The polymerizable composition of the present invention exhibits high curability not only when photocured using, for example, a halogen lamp-curing unit that emits dental visible light but also when photocured using an LED light-curing unit. Accordingly, the polymerizable composition of the present invention has a high cure rate at the time of photocuring, and the thickness of its unpolymerized portion is small. The polymerizable composition of the present invention is particularly useful for dental applications. When it is used for a dental adhesive material, a cured product thereof exhibits high bond strength to a tooth structure. The polymerizable composition of the present invention hardly has discoloration and adhesive properties deterioration even when stored for a long time, so that it is excellent storage stability.

### DESCRIPTION OF EMBODIMENTS

In polymerizable compositions that exhibit curability when irradiated with light α-diketone typified by camphorquinone, and, if needed, tertiary amine, conventionally have been used for a photopolymerization initiator composition. However, camphorquinone has a disadvantage in that it is too yellowish and makes it difficult to adjust the color of a dental material when contained therein. Because of this, acylphosphine oxides have been used instead of camphorquinone in recent years (see Patent Literature 3 mentioned above, for example). In view of the foregoing, the present inventors made intensive studies, and as a result, they found that, in the case of photocuring using an LED light-curing unit, it is very effective for a polymerizable composition to contain a photopolymerization initiator composition obtained by adding a specific amount of α-diketone, such as camphorquinone, to bisacylphosphine oxides and tertiary amine.

First, the photopolymerization initiator composition (hereinafter referred to as a photopolymerization initiator composition (I)) contained in the polymerizable composition of the present invention will be described below. The photopolymerization initiator composition (1) contains: a photopolymerization initiator component consisting of only a bisacylphosphine oxide (A) and α-diketone (B); and tertiary amine (C).

### Bisacylphosphine oxide (A)

An example of the bisacylphosphine oxide (A) used in the present invention is a compound represented by formula (1) below.

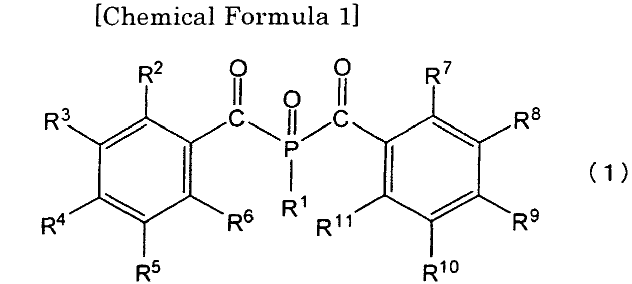

In the above formula, R¹ denotes an alkyl group, alkenyl group, alkynyl group, aryl group, alkoxy group, acyl group, or acyloxy group, and R² to R¹¹ each denote independently a hydrogen atom, halogen atom, alkyl group, alkenyl group, alkynyl group, aryl group, alkoxy group, acyl group, or acyloxy group.

Alkyl groups having 1 to 10 carbon atoms are preferable as the alkyl groups denoted as R¹ to R¹¹, and examples thereof include a methyl group, ethyl group, n-propyl group, isopropyl group, cyclopropyl group, n-butyl group, isobutyl group, sec-butyl group, tert-butyl group, cyclobutyl group, n-pentyl group, isopentyl group, neopentyl group, tert-pentyl group, 2,4,4-trimethylpentyl group, cyclopentyl group, n-hexyl group, isohexyl group, cyclohexyl group, n-heptyl group, cycloheptanyl group, n-octyl group, 2-ethylhexyl group, cyclooctanyl group, n-nonyl group, cyclononanyl group, and n-decyl group.

Alkenyl groups having 2 to 10 carbon atoms are preferable as the alkenyl groups denoted as R¹ to R¹¹, and examples thereof include a vinyl group, allyl group, methylvinyl group, propenyl group, butenyl group, pentenyl group, hexenyl group, cyclopropenyl group, cyclobutenyl group, cyclopentenyl group, and cyclohexenyl group.

Alkynyl groups having 2 to 10 carbon atoms are preferable as the alkynyl groups denoted as R¹ to R¹¹, and examples thereof include an ethynyl group, 1-propynyl group, 2-propynyl group, 1-butynyl group, 1-methyl-2-propynyl group, 2-butynyl group, 3-butynyl group, 1-pentynyl group, 1-ethyl-2-propynyl group, 2-pentynyl group, 3-pentynyl group, 1-methyl-2-butynyl group, 4-pentynyl group, 1-methyl-1-3-butynyl group, 2-methyl-3-butynyl group, 1-hexynyl group, 2-hexynyl group, 1-ethyl-2-butynyl group, 3-hexynyl group, 1-methyl-2-pentynyl group, 1-methyl-1-3-pentynyl group, 4-methyl-1-pentynyl group, 3-methyl-1-pentynyl group, 5-hexynyl group, and 1-ethyl-3-butynyl group.

Aryl groups having 6 to 30 carbon atoms are preferable as the aryl groups denoted as R¹ to R¹¹, and examples thereof include a phenyl group, o-tolyl group, m-tolyl group, p-tolyl group, xylyl group, mesityl group, naphthyl group, and anthracenyl group. These aryl groups further may include an alkyl group, alkoxy group, alkenyl group, and alkynyl group.

Alkoxy groups having 1 to 10 carbon atoms are preferable as the alkoxy groups denoted as R¹ to R¹¹, and examples thereof include a methoxy group, ethoxy group, n-propoxy group, isopropoxy group, cyclopropyropoxy group, n-butoxy group, isobutoxy group, sec-butoxy group, tert-butoxy group, cyclobutoxy group, and cyclohexyloxy group.

Acyl groups having 1 to 10 carbon atoms are preferable as the acyl groups denoted as R¹ to R¹¹, and examples thereof include: a formyl group; an alkylcarbonyl group having 2 to 10 carbon atoms, such as an acetyl group, propionyl group, butyryl group, isobutyryl group, valeryl group, pivaloyl group, hexanoyl group, heptanoyl group, octanoyl group, and decenoyl group; an arylcarbonyl group having 7 to 10 carbon atoms, such as a benzoyl group; and an arylalkylcarbonyl group, having 8 to 10 carbon atoms, such as a benzylcarbonyl group.

Acyloxy groups having 1 to 10 carbon atoms are preferable as the acyloxy groups denoted as R¹ to R¹¹, and examples thereof include groups, in which one oxygen atom is bonded to each of the groups described above as examples of the acyl groups.

Examples of halogen atoms denoted as R² to R¹¹ include a fluorine atom, chlorine atom, and bromine atom. Among these, a chlorine atom is preferable.

An alkyl group and aryl group each are preferable as R¹. A hydrogen atom, halogen atom, alkyl group, and alkoxy group are preferable as R² to R¹¹.

Examples of the bisacylphosphine oxide (A) represented by the formula (1) include bis-(2.6-clichlorobenzoyl)phenylpbosphine oxide, bis-(2;6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, bis-(2,6-dimethoxybenzoyl)phenylphosphine oxide, bis-(2,6-dimethoxybenzoyl)-2,4,4-trimethylpentylphosphine oxide, bis-(2,6-dimethoxybenzoyl)-2,5-dimethylphenylphosphine oxide, bis-(2,4,6-trimethylbenzoyl)phenylphosphine oxide, and (2,5,6-trimethylbenzoyl)-2,4,4-trimethylpentylphosphine oxide. Among these, bis-(2,4,6-trimethylbenzoyl)phenylphosphine oxide is most preferable from the viewpoint of photocurability and adhesive properties when contained in the polymerizable composition.

### α-diketone (B)

Examples of the α-diketone (B) used in the present invention include diacetyl, dibenzyl, camphorquinone, 2,3-pentadione, 2,3-octadione, 9,10-phenanthrenequinone, 4,4'-oxybenzyl, and acenaphthenequinone. Among these, camphorquinone is particularly preferable from the viewpoint of having the maximum absorption wavelength in the visible light range.

The photopolymerization initiator component of the photopolymerization initiator composition (I) consists of only the bisacylphosphine oxide (A) and the α-diketone (B), and contains no other photopolymerization initiators. Limiting the photopolymerization initiator component of the photopolymerization initiator composition (I) to these photopolymerization initiators makes it possible to produce a polymerizable composition that is free from discoloration during storage and has excellent stability to environment light such as fluorescent light and dental light.

### Tertiary amine (C)

The tertiary amine (C) used in the present invention may be aliphatic amine or aromatic amine.

Examples of tertiary aliphatic amine include N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. Among these, N-methyldiethanolamine and triethanolamine are used more preferably from the viewpoint of curability and storage stability of the composition.

Examples of aromatic amines include N,N-bis(2-hydroxyethyl)-3,5-dimethylaniline; N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, 4-N,N-dimethylaminobenzoic acid ethyl ester, 4-N,N-dimethylaminobenzoic acid methyl ester, N,N-dimethylaminobenzoic acid n-butoxyethyl ester, 4-N,N-dimethylaminobenzoic acid 2-(methacryloyloxy)ethyl ester, 4-N,N-dimethylaminobenzophenone, and butyl 4-dimethylaminobenzoate. Among these, at least one selected from the group consisting of N,N-di(2-hydroxyethyl)-p-toluidine, 4-N,N-dimethylaminobenzoic acid ethyl ester, N,N-dimethylaminobenzoic acid n-butoxyethyl ester, and 4-N,N-dimethylaminobenzophenone is used preferably from the viewpoint of being capable of providing the polymerizable composition with excellent curability.

Next, the amounts of the bisacylphosphine oxide (A), the α-diketone (B) and the tertiary amine (C) to be added will be described. In the photopolymerization initiator composition (I), a weight ratio (A):(B) between the bisacylphosphine oxide (A) and the α-diketone (B) is 1:9 to 9:1. When the amounts of the bisacylphosphine oxide (A) and the α-diketone (B) to be added are out of this range, the adhesive properties to a tooth structure is deteriorated. Preferably, the weight ratio (A):(B) is 1:5 to 5:1.

In the photopolymerization initiator composition (I), a weight ratio (B):(C) between the α-diketone (B) and the tertiary amine (C) is preferably 1:10 to 5:1, and more preferably 1:5 to 4:1. A content of the tertiary amine (C) exceeding these ratios may cause insufficient photocurability. A content of the tertiary amine (C) below these ratios also may cause insufficient photocurability because of a reduced amount of radicals generated.

The photopolymerization initiator composition (I) may contain other components, such as a solvent and a thermal polymerization inhibitor, as long as they do not impair the advantageous effects of the present invention. However, for the sake of photopolymerization, the photopolymerization initiator composition (I) contains no other photopolymerization initiators because the combination of the components (A) to (C) allows it to exhibit satisfactory polymerization initiation ability both with an LED light source and a halogen lamp light source. Thus, it is preferable that in the photopolymerization initiator composition (I), a component involved in photopolymerization consists essentially of the bisacylphosphine oxide (A), the α-diketone (B), and the tertiary amine (C). Here, the "component involved in photopolymerization" indicates the photopolymerization initiator component and the polymerization accelerator component of the photopolymerization initiator. The photopolymerization initiator composition (I) can be prepared by mixing the components (A) to (C) together according to a conventional method.

The polymerizable composition of the present invention containing the photopolymerization initiator composition (I) and the polymerizable monomer (D) exhibits high curability not only in the case of using a halogen lamp light source but also in the case of using an LED light source, and exhibits high adhesive properties to a tooth structure when applied to dental applications.

In the present invention, the terms "halogen lamp light source" and "halogen lamp-curing unit," mean a halogen lamp light source and a halogen lamp-curing unit that emit dental visible light, respectively, or a halogen lamp light source and a halogen lamp-curing unit that have similar light emitting property (emission spectrum) to dental visible light, respectively. The terms "LED light source" and "LED light-curing unit" also should be understood in the same way.

Next, the polymerizable monomer (D) contained in the polymerizable composition of the present invention will be described.

### Polymerizable monomer (D)

The polymerizable monomer (D) can be used without limitation as long as it is allowed to start polymerization by the photopolymerization initiator composition (I). The polymerizable composition of the present invention is used mainly for dental applications. From the viewpoint of usefulness in the just-mentioned applications, the polymerizable monomer (D) is preferably at least one polymerizable monomer selected from the group consisting of: a polymerizable monomer (d-1) having an unconjugated carbon chain with at least four carbon atoms bonded continuously, at least two polymerizable groups, and at least two hydroxyl groups; a polymerizable monomer (d-2) having one polymerizable group and at least one hydroxyl group; a polymerizable monomer (d-3) having an acidic group; and a crosslinkable polymerizable monomer (d-4) other than the polymerizable monomer (d-1). Particularly it is preferable that the polymerizable monomer (D) contain the polymerizable monomer (d-1) from the viewpoint of curability and adhesive properties to a tooth structure. By allowing the polymerizable monomer (D) to contain at least two types of the polymerizable monomers, it is possible to provide the polymerizable composition with advanced features.

### Polymerizable monomer (d-1)

The polymerizable monomer (d-1) has an unconjugated carbon chain with at least four carbon atoms bonded continuously, at least two polymerizable groups, and at least two hydroxyl groups. For the polymerizable monomer (d-1), polymerizable monomers that satisfy such a definition can be used independently or two or more of them can be used in combination.

The polymerizable monomer (d-1) has crosslinkability because the number of the polymerizable groups is two or more. A polymerizable composition containing this polymerizable monomer has high curability, and a cured product thereof has high mechanical strength. Furthermore, since the polymerizable monomer (d-1) has at least two hydroxyl groups, a polymerizable composition containing this polymerizable monomer has high penetrability into a collagen layer of dentin and enhanced adhesive properties to a tooth structure.

With respect to the polymerizable monomer (d-1), the polymerizable group denotes a group including a radical polymerizable functional group and examples thereof include a group including a vinyl group. Particularly, from the viewpoint of polymerization reactivity, the group represented by following formula (3), (4), or (5) below is preferable as the polymerizable group. Among these, from the viewpoint of ease of introduction into the polymerizable monomer (d-1), a group represented by the formula (3) is most preferable.

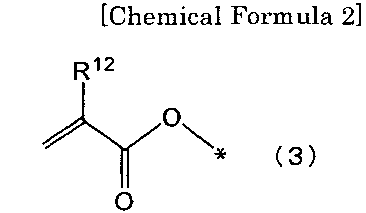

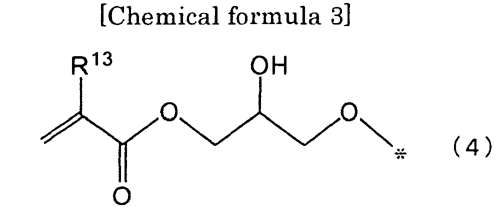

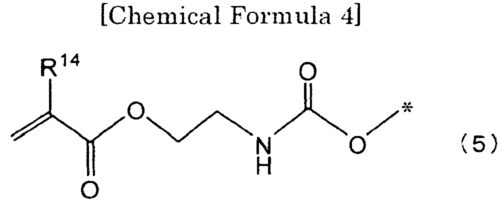

In the above formulae, R¹², R¹³, and R¹⁴ each denote a hydrogen atom or an aliphatic hydrocarbon group having 1 to 10 carbon atoms, and "*" denotes a bond. Examples of the aliphatic hydrocarbon group having 1 to 10 carbon atoms include an alkyl group having 1 to 10 carbon atoms, an alkenyl group having 2 to 10 carbon atoms, and an alkynyl group having 2 to 10 carbon atoms.

The alkyl group having 1 to 10 carbon atoms may be any one of linear, branched, and cyclic, and examples thereof include a methyl group, ethyl group, n-propyl group, isopropyl group, cyclopropyl group, n-butyl group, isobutyl group, sec-butyl group, tert-butyl group, cyclobutyl group, n-pentyl group, isopentyl group, neopentyl group, tert-pentyl group, cyclopentyl group, n-hexyl group, isohexyl group, cyclohexyl group, n-heptyl group, cycloheptanyl group, n-octyl group, 2-ethylhexyl group, cyclooctanyl group, n-nonyl group, cyclononanyl group, and n-decyl group.

The alkenyl group having 2 to 10 carbon atoms may be any one of linear, branched, and cyclic, and examples thereof include a vinyl group, allyl group, methylvinyl group, propenyl group, butenyl group, pentenyl group, hexenyl group, cyclopropenyl group, cyclobutenyl group, cyclopentenyl group, and cyclohexenyl group.

The alkynyl group having 2 to 10 carbon atoms may be any one of linear, branched, and cyclic, and examples thereof include an ethynyl group, 1-propynyl group, 2-propynyl group, 1-butynyl group, 1-methyl-2-propynyl group, 2-butynyl group, 3-butynyl group, 1-pentynyl group, 1-ethyl-2-propynyl group, 2-pentynyl group, 3-pentynyl group, 1-methyl-2-butynyl group, 4-pentynyl group, 1-methyl-3-butynyl group, 2-methyl-3-butynyl group, 1-hexynyl group, 2-hexynyl group, 1-ethyl-2-butynyl group, 3-hexynyl group, 1-methyl-2-pentynyl group, 1-methyl-3-pentynyl group, 4-methyl-1-pentynyl group, 3-methyl-1-pentynyl group, 5-hexynyl group, and 1-ethyl-3-butynyl group.

When the polymerizable monomer (d-1) is used in, for example, dental applications, radical polymerization is performed. Accordingly, it is preferable that R¹², R¹³ and R¹⁴ each be a hydrogen atom or a methyl group from the viewpoint of radical polymerization reactivity of the final product. Furthermore, when the polymerizable monomer (d-1) is used in a dental composition, the polymerizable group may be detached from the polymerizable monomer (d-1) by, for example, hydrolysis. When the stimulativeness of the detached polymerizable group to a biological body is taken into account, it is preferable that the polymerizable group include a methacryloyloxy group. Therefore, it is more preferable that R¹², R¹³, and R¹⁴ each be a methyl group.

The polymerizable monomer (d-1) includes at least two polymerizable groups, and the at least two polymerizable groups may be identical to or different from each other.

The polymerizable monomer (d-1) has an unconjugated carbon chain with at least four carbon atoms bonded continuously. Preferably, this carbon chain composes the whole or a part of the skeleton of the polymerizable monomer (d-1), and the polymerizable groups and hydroxyl groups are bonded to the carbon chain.

An example of the polymerizable monomer (d-1) is a compound in which a part of hydroxyl groups of a tetravalent or more alcohol compound having an unconjugated carbon chain with at least four carbon atoms bonded continuously is substituted by polymerizable groups so that the number of each of the hydroxyl groups and the polymerizable groups is two or more. Examples of the tetravalent or more alcohol compound having an unconjugated carbon chain with at least four carbon atoms bonded continuously are not particularly limited. Preferable examples thereof include sugar alcohol, monosaccharides, disaccharides, and trisaccharides, each of which has 4 to 20 carbon atoms. Examples of the sugar alcohol that is used preferably include erythritol, a sugar alcohol having 4 carbon atoms, xylitol, ribitol, and arabinitol, each of which is a sugar alcohol having 5 carbon atoms, mannitol, sorbitol, and iditol, each of which is a sugar alcohol having 6 carbon atoms, and maltitol, a sugar alcohol having 12 carbon atoms. Furthermore, glucamine, a sugar alcohol containing an amino group, also is used preferably. Examples of monosaccharides that are used preferably include xylose, ribose, arabinose, and lyxose, each of which is a monosaccharide having 5 carbon atoms, as well as glucose, mannose, galactose, sorbose, and fructose, each of which is a monosaccharide having 6 carbon atoms. Furthermore, glucosamine, mannosamine, galactosamine, N-acetylglucosamine, N-acetylmannosamine, and N-acetylgalactosamine that are monosaccharides, each of which contains an amino group and a derivative thereof, also are used preferably. Examples of disaccharides that are used preferably include trehalose, sucrose, maltose, lactose, and cellobiose. Examples of trisaccharides that are used preferably include Coupling Sugar (registered trademark), lactosucrose, maltotriose, and isomaltotriose. The polymerizable composition of the present invention is used preferably as a dental composition and more preferably as a dental adhesive composition. From the viewpoint of adhesive properties to a tooth structure (particularly dentin), it is preferable that it have high penetrability into a tooth structure (particularly dentin). From such a viewpoint, the carbon number of the aforementioned alcohol compound is more preferably 4 to 15, further preferably 4 to 9, and particularly preferably 4 to 7. Moreover, from the same viewpoint, the number of the hydroxyl groups of the alcohol compound is preferably 4 to 15, more preferably 4 to 9, and particularly preferably 4 to 7. Specific examples of preferable alcohol compounds include: erythritol, mannitol, sorbitol, and maltitol as sugar alcohols; glucose and glucosamine as monosaccharides; trehalose and maltose as disaccharides; and maltotriose as trisaccharides. Among these, erythritol, mannitol, glucose, and trehalose are further preferable and erythritol and mannitol are particularly preferable.

Preferably, the polymerizable monomer (d-1) has a group represented by formula (6) below. This group is a structure that is characteristic to the compounds indicated above as examples.

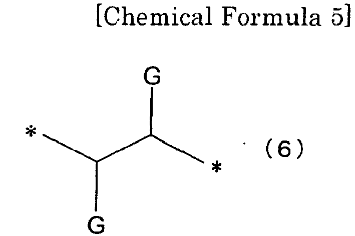

In the above formula, G denotes a hydroxyl group or polymerizable group and "*" denotes a bond.

With respect to the structure of the polymerizable monomer (d-1), specifically, the polymerizable monomer (d-1) is preferably a compound represented by formula (7).

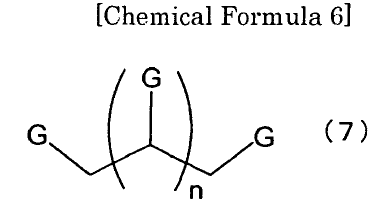

In the above formula, Gs are hydroxyl groups or polymerizable groups, n is an integer of 2 or more, at least two of the Gs are hydroxyl groups, and at least two of the Gs are polymerizable groups.

In this structure, the polymerizable group or the hydroxyl group is bonded to each carbon atom of the carbon chain, and therefore the polymerizable groups and hydroxyl groups are closely-located with a high density. Accordingly, when a polymerizable composition containing the polymerizable monomer (d-1) is applied as a dental material, it exhibits excellent curability and adhesive properties. Furthermore, it also has an advantage that it can be produced easily using sugar alcohol.

From the viewpoints of curability and adhesive properties to a tooth structure of the polymerizable composition as well as availability of raw materials, n is preferably an integer of 2 to 18, more preferably an integer of 2 to 9, and most preferably an integer of 2 to 4.

When curability of the polymerizable composition is considered important, the polymerizable monomer (d-1) is preferably a compound represented by formula (2) below.

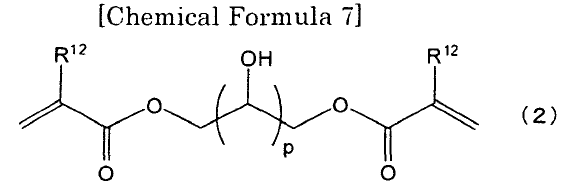

In the above formula, R¹² denotes the same as described above, and p denotes an integer of 2 or more.

The compound represented by the formula (2) has polymerizable groups represented by the formula (3) at the both ends of an unconjugated carbon chain with at least four carbon atoms bonded continuously and has particularly high polymerization performance due to a steric factor. Accordingly, when a polymerizable composition containing a compound represented by the formula (2) is applied as a dental application, it serves as a composition with particularly high curability. Moreover, since it has a plurality of hydroxyl groups, it has excellent penetrability into a collagen layer of dentin as well as excellent adhesive properties to a tooth structure.

p is preferably 2 to 4. This is because when a decomposition product is produced by an action such as hydrolysis inside an oral cavity, the decomposition product is a highly safe compound such as erythritol, xylitol, sorbitol, or mannitol. Examples of compounds in which p is 2 to 4 include erythritol di(meth)acrylate, xylitol di(meth)acrylate, and sorbitol di(meth)acrylate. Furthermore, erythritol di(meth)acrylate in which p is 2 is more preferable, and when the aforementioned viewpoints of polymerizability and stimulativeness to a biological body also are taken into consideration, erythritol dimethacrylate represented by general formula (8) below is most preferable.

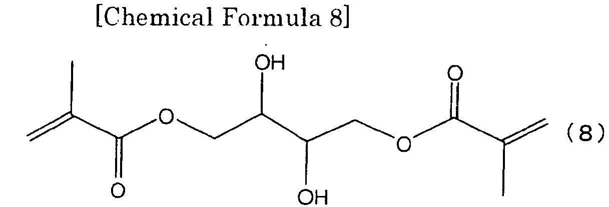

On the other hand, when adhesive properties to a tooth structure of the polymerizable composition is considered important, the polymerizable monomer (d-1) is preferably a compound represented by formula (9) below.

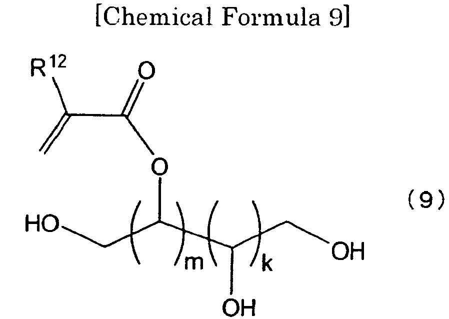

In the above formula, R¹² denotes the same as described above, m denotes an integer of 2 or more, k denotes an integer of 1 or more, and the sequence order of m units having an ester group and k units having a hydroxyl group is arbitrary.

From the viewpoints of curability and adhesive properties to a tooth structure of the polymerizable composition as well as availability of raw materials, m is preferably 2 to 5, more preferably 2 to 4, and most preferably 2. Furthermore, k is preferably 1 to 5, more preferably 2 to 4, and most preferably 2. The total of m and k is preferably 3 to 18, more preferably 3 to 9, further preferably 4 to 8, and most preferably 4.

The compound represented by formula (9) has at least three hydroxyl groups, two of which are primary hydroxyl groups. These primary hydroxyl groups are highly advantageous for interaction with a tooth structure (particularly dentin). Accordingly, when a polymerizable composition containing a compound represented by formula (9) is applied as dental applications, a composition with particularly high adhesive properties to a tooth structure (particularly dentin) is obtained. Moreover, since at least two groups represented by formula (3) are included as polymerizable groups, curability also is high.

Furthermore, among the compounds represented by formula (9), compounds represented by formulae (10) and (11) are preferable from the viewpoints of curability and adhesive properties to a tooth structure of the polymerizable composition.

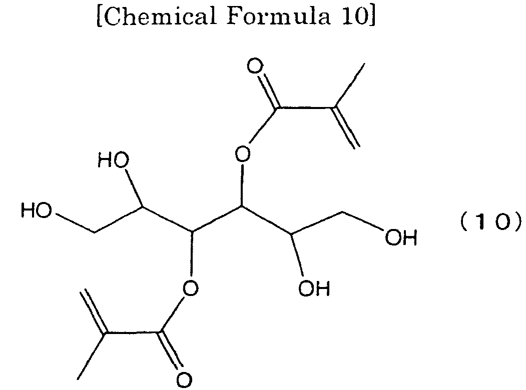

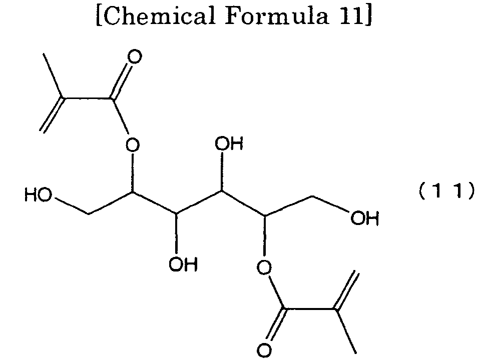

The polymerizable monomer (d-1) can be obtained through production by a known method. Specifically, for example, any one of carboxylic acid having a polymerizable group (for example, a compound represented by formula (12)) or a derivative thereof, an epoxy compound having a polymerizable group (for example, a compound represented by formula (13)), and an isocyanate compound having a polymerizable group (for example, a compound represented by formula (14)) may be reacted with a tetravalent or more alcohol compound having an unconjugated carbon chain with at least four carbon atoms bonded continuously according to a conventional method and this may then be purified by a separation means such as chromatography. In order to improve the yield, the esterification reaction may be carried out after the carboxylic acid having a polymerizable group is converted into a derivative such as an acid halide.

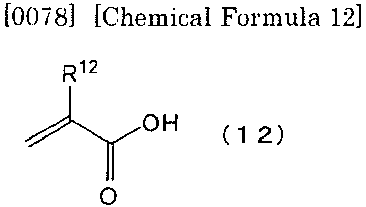

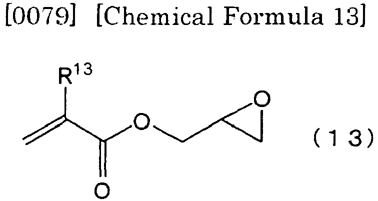

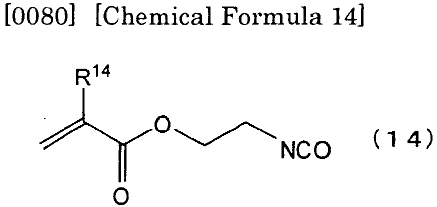

When the polymerizable monomer (d-1) is particularly a compound represented by formula (9), especially a compound represented by formula (10) or (11), it is preferable that the polymerizable monomer (d-1) be produced by performing a step (a) where using a compound in which primary hydroxyl groups of the alcohol compound are protected beforehand, as a raw material, the compound and a carboxylic acid having a polymerizable group (in this case, a compound represented by the formula (12)) or a derivative thereof are esterified, and a step (b) where the protecting groups of the primary hydroxyl groups of the resultant ester compound are deprotected. The derivative of the carboxylic acid having a polymerizable group is not particularly limited but an acid halide or acid anhydride is used preferably". When the reactivity with the alcohol compound is taken into account, an acid halide is used more preferably. Furthermore, among the acid halides, acid chloride is used particularly preferably when availability and storage stability of the compound are taken into account. The production process including these steps allows a polymerizable monomer to be obtained with high yield and therefore is suitable for industrial production.

The compound in which primary hydroxyl groups of the alcohol compound are protected beforehand can be obtained as a commercially available product, for example, 1,2:5,6-di-O-isopropylidene-D-mannitol and 1,3:4,6-di-O-benzylidene-D-mannitol. Furthermore, it also can be produced by carrying out a step of protecting the primary hydroxyl groups of the alcohol compound. In a compound in which primary hydroxyl groups of the alcohol compound are protected beforehand, it is preferable that a part of hydroxyl groups other than the primary hydroxyl groups be protected while a plurality of hydroxyl groups are allowed to remain. In this manner, a structure having at least three hydroxyl groups is obtained easily.

The step of protecting the primary hydroxyl groups of the alcohol compound can be carried out by performing a known reaction for introducing protecting groups.

It is advantageous to select a group that is introduced preferentially into a primary hydroxyl group, as a protecting group for the primary hydroxyl groups of the alcohol compound. Furthermore, for the protecting group, it is advantageous to select one that tends not to undergo a deprotection reaction during the esterification reaction and tends not to allow the ester bond to be cleaved during the deprotection reaction. From these viewpoints, protecting groups that are used preferably are ether protecting groups, silyl ether protecting groups, and acetal protecting groups. Ether protecting groups that are used more preferably are a 1-ethoxyethyl ether group and triphenylmethyl ether group. Silyl ether protecting groups that are used more preferably are a trilsopropylsilyl ether group, t-butyldimethylsilyl ether group, and t-butyldiphenylsilyl ether group. Each of these protecting groups can be introduced preferentially into a primary hydroxyl group and can be deprotected under a mild acidic condition, and therefore it has an advantage that deprotection can be achieved without cleaving the ester bond. On the other hand, acetal protecting groups that are used more preferably are an isopropylidene group, cycloheptylidene group, benzylidene group, and p-methoxybenzylidene group. When using an acetal protecting group, it not only can be introduced preferentially into a primary hydroxyl group but also can protect two or more hydroxyl groups including the primary hydroxyl group at the same time. Accordingly the acetal protecting group is especially suitable for synthesis of the polymerizable monomer (d-1). Therefore, among the ether protecting groups, silyl ether protecting groups, and acetal protecting groups, the acetal protecting groups are used further preferably. Moreover, from the viewpoints that deprotection is possible under a particularly mild acidic condition and a byproduct produced at the time of deprotection can be removed easily, an isopropylidene group is used particularly preferably.

The step of esterifying a compound in which primary hydroxyl groups of the alcohol compound are protected beforehand and a carboxylic acid having a polymerizable group or a derivative thereof can be carried out according to a known method. For the esterification reaction, it is important to select suitable reaction conditions (particularly, the temperature condition and the type of catalyst) under which a deprotection reaction tends not to occur, with consideration given to the type of the protecting group. Furthermore, it is important to select the reaction conditions (particularly, the amounts of the compound in which primary hydroxyl groups of the alcohol compound are protected beforehand and the carboxylic acid having a polymerizable group or a derivative thereof to be used) so that after the esterification reaction, a plurality of ester bonds are formed and the total number of the protected hydroxyl groups and unreacted hydroxyl groups is at least three, in one molecule.

The step of deprotecting the protecting groups of the primary hydroxyl groups of the resultant ester compound may be carried out according to a known method depending on the type of the protecting group. In this case, it is important to select reaction conditions (particularly, the temperature condition and the type of catalyst) under which the ester bond tends not to be cleaved. As described above, when the ether protecting groups, silyl ether protecting groups, and acetal protecting groups that are preferable as the protecting group of the primary hydroxyl group are used, all of them can be deprotected under mild acidic conditions and therefore deprotection can be performed without allowing the ester bond to be cleaved. Furthermore, the silyl ether protecting groups can be deprotected with extremely high selectivity by the use of a fluorine-containing compound such as TBAF (tetrabutylammonium fluoride) and thus are highly useful. In the case of deprotection under an acidic condition, for example, mineral acids such as hydrochloric acid and sulfuric acid and aqueous solutions thereof; organic acids such as formic acid, acetic acid, and trifluoroacetic acid and aqueous solutions thereof; and cation exchange resin are used preferably. Among these, since the acidity is suitable and deprotection can be performed with cleavage of the ester bond being prevented efficiently, organic acids such as formic acid, acetic acid, and trifluoroacetic acid and aqueous solutions thereof are more preferable, and formic acid, acetic acid, and aqueous solutions thereof are further preferable.

Since the polymerizable monomer (d-1) has a plurality of polymerizable groups and a plurality of hydroxyl groups, it is excellent in crosslinking reactivity and can interact strongly with a compound having a hydrophilic group. Accordingly, when the polymerizable monomer is mixed with a suitable component into a polymerizable composition, the composition thus obtained exhibits excellent curability and adhesive properties in various applications including dental applications. From the viewpoint of obtaining both curability and adhesive properties to a tooth structure of the polymerizable composition in a balanced manner, compounds represented by the aforementioned formulae (2) and (9) can be used in combination.

The amount of polymerizable monomer (d-1) to be added may be determined suitably according to the application of the polymerizable composition. Preferably, 1 to 99 parts by mass of the polymerizable monomer (d-1) is contained in 100 parts by mass of the polymerizable monomer (D). When a polymerizable composition in which the amount of the polymerizable monomer (d-1) to be added is in such a range is used as a dental composition, there are advantages that penetrability into a collagen layer of dentin is excellent and bond strength is high. When the amount of the polymerizable monomer (d-1) to be added is less than 1 part by mass, bond strength may be reduced and bond durability also may be reduced. Therefore, the amount is more preferably at least 2 parts by mass and further preferably at least 5 parts by mass. On the other hand, the amount of the polymerizable monomer (d-1) to be added exceeding 99 parts by mass results in insufficient decalcification and sufficiently high bond strength may not be obtained. Therefore the amount is more preferably 98 parts by mass or less and further preferably 95 parts by mass or less.

In the following description, terms "monofunctional", "bifunctional", and "trifunctional" are used and the terms "monofunctional", "bifunctional", and "trifunctional" indicate that one, two, and three polymerizable groups each are contained in one molecule. Preferably, the polymerizable groups included in the below-mentioned polymerizable monomers are groups that are radical-copolymerizable with polymerizable groups of the polymerizable monomer (d-1).

### Polymerizable monomer (d-2) having one polymerizable group and at least one hydroxyl group

Preferably, the polymerizable monomer (D) contains the polymerizable monomer (d-2) having one polymerizable group and at least one hydroxyl group. Since the polymerizable monomer (d-2) has at least one hydroxyl group, it has high hydrophilicity. Accordingly, when a polymerizable composition containing the polymerizable monomer (d-2) is used as a dental composition, its penetrability into a collagen layer of dentin is enhanced and thereby high bond strength are obtained. Since the polymerizable monomer (d-2) has a polymerizable group, not only radical polymerization can occur but also copolymerization with another monomer can occur. From the viewpoint of ease of radical polymerization, the polymerizable group of the polymerizable monomer (d-2) is preferably a (meth)acrylic group or (meth)acrylamide group. The polymerizable monomer (d-2) is used preferably as a component of a dental composition. However, since the inside of an oral cavity has a humid environment, the polymerizable group may be detached by, for example, hydrolysis. When consideration is given to stimulativeness of a detached polymerizable group to a biological body, the polymerizable group of the polymerizable monomer (d-2) is preferably a methacrylic group or methacrylamide group.

The polymerizable monomers (d-2) can be used independently or two or more of them can be used in suitable combination. The polymerizable monomer (d-2) is not particularly limited, but examples thereof include 2-hydroxyethyl(meth)acrylate, 3-hydroxypropyl(meth)acrylate, 4-hydroxybutyl(meth)acrylate, 6-hydroxyhexyl(meth)acrylate, 10-hydroxydecyl(meth)acrylate, propylene glycol mono(meth)acrylate, glycerol mono(meth)acrylate, erythritol mono(meth)acrylate, N-methylol(meth)acrylamide, N-hydroxyethyl(meth)acrylamide, and N,N-(dihydroxyethyl)(meth)acrylamide. Among these, from the viewpoint of improving the penetrability into a collagen layer of dentin, 2-hydroxyethyl(meth)acrylate, 3-hydroxypropyl(meth)acrylate, glycerol mono(meth)acrylate, and erythritol mono(meth)acrylate are preferable and 2-hydroxyethylmethacrylate is particularly preferable.

The amount of the polymerizable monomer (d-2) to be added is not particularly limited, but it is preferable that 1 to 90 parts by mass of polymerizable monomer (d-2) be contained in 100 parts by mass of the polymerizable monomer (D). When a polymerizable composition in which the amount of the polymerizable monomer (d-2) to be added is in such a range is used as a dental composition, both high penetrability into a collagen layer of dentin and high bond strength are obtained. When the amount of the polymerizable monomer (d-2) to be added is less than 1 part by mass, contribution of the polymerizable monomer (d-2) to penetration into a collagen layer of dentin may not be obtained and the bond strength may be reduced. The amount of the polymerizable monomer (d-2) to be added is more preferably at least 3 parts by mass, further preferably at least 5 parts by mass, and particularly preferably at least 7 parts by mass. On the other hand, when the amount of the polymerizable monomer (d-2) to be added exceeds 90 parts by mass, sufficiently high curability cannot be obtained and therefore the mechanical strength of the cured product may be reduced. Accordingly, the bond strength may be reduced. The amount of the polymerizable monomer (d-2) to be added is more preferably 80 parts by mass or less, further preferably 75 parts by mass or less, and particularly preferably 70 parts by mass or less.

### Polymerizable monomer (d-3) having acidic group

Preferably, the polymerizable monomer (D) contains the polymerizable monomer (d-3) having an acidic group. When a polymerizable composition containing the polymerizable monomer (d-3) having an acidic group is used as a dental composition, the polymerizable monomer (d-3) itself that has an acidic group has an acid etching effect and a primer treatment effect. Therefore, it has advantages that, for example, pretreatments such as an acid etching treatment and a primer treatment are not necessary. Accordingly, use of the polymerizable monomer (d-3) having an acidic group makes it possible to provide a bonding material that is simple to use and has high bond strength and excellent bond durability, particularly preferably a one-component bonding material.

The polymerizable monomers (d-3) having an acidic group can be used independently or two or more of them can be used in suitable combination. The polymerizable monomer (d-3) having an acidic group is not particularly limited. Examples thereof include a monofunctional polymerizable monomer having one carboxyl group or an acid anhydride group thereof in the molecule, a monofunctional polymerizable monomer having a plurality of carboxyl groups or an acid anhydride group thereof in the molecule, and a monofunctional polymerizable monomer having a phosphinyloxy group or phosphonooxy group in the molecule (also referred to as a monofunctional radical polymerizable phosphoric acid ester).

Examples of the monofunctional polymerizable monomer having one carboxyl group or an acid anhydride group thereof in the molecule include (meth)acrylic acid, N-(meth)acryloylglycine, N-(meth)acryloylaspartic acid, N-(meth)acryloyl-5-aminosalicylic acid, 2-(meth)acryloyloxyethyl hydrogen succinate, 2-(meth)acryloyloxyethyl hydrogen phthalate, 2-(meth)acryloyloxyethyl hydrogen malate, 6-(meth)acryloyloxyethyl naphthalene-1,2,6-tricarboxylic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoicacid, 3-(meth)acryloyloxybenzoic acid, 4-(meth)acryloyloxybenzoic acid, N-(meth)acryloyl-4-aminosalicylic acid, and compounds obtained by converting the carboxyl group of these compounds into an acid anhydride group.

Examples of the monofunctional polymerizable monomer having a plurality of carboxyl groups or an acid anhydride group thereof in the molecule include 11-(meth)acryloyloxyundecane-1,1-dicarboxylic acid, 10-(meth)acryloyloxydecane-1,1-dicarboxylic acid, 12-(meth)acryloyloxydodecane-1,1-dicarboxylic acid, 6-(meth)acryloyloxyhexane-1,1-dicarboxylic acid, 2-(meth)acy-loyloxyethyl-3'-methacryloyloxy-2'-(3,4-dicarboxybenzoyloxy)propyl succinate, 4-(2-(meth)acryloyloxyethyl)trimeritate anhydride, 4-(2-(meth)acryloyloxyethyl)trimeritate, 4-(meth)acryloyloxyethyl trimeritate, 4-(meth)acryloyloxybutyl trimeritate, 4-(meth)acryloyloxyhexyl trimeritate, 4-(meth)acryloyloxydecyl trimeritate, 6-(meth)acryloyloxyethylnaphthalene-1,2,6-tricarboxylic acid anhydride, 6-(meth)acryloyloxyethylnaphthalene-2,3,6-tricarboxylic acid anhydride, 4-(meth)acryloyloxyethylcarbonylpropionoyl-1,8-n'aphthalic acid anhydride, 4-(meth)acryloyloxyethylnaphthalene-1,8-tricarboxylic acid anhydride, 9-(meth)acryloyloxynonane-1,1-dicarboxylic acid, 13-(meth)aciyloyloxytridecane-1,1-dicarboxylic acid, and 11-(meth)acrylamideundecane-1,1-dicarboxylic acid.

Examples of the monofunctional polymerizable monomer having a phosphinyloxy group or phosphonooxy group in the molecule (also referred to as a monofunctional radical polymerizable phosphoric acid ester) include 2-(meth)acryloyloxyethyl dihydrogenphosphate, 2-(meth)acryloyloxyethylphenyl hydrogenphosphate, 10-(meth)acryloyloxydecyl dihydrogenphosphate, 6-(meth)acryloyloxyhexyl dihydrogenphosphate, 2-(meth)acryloyloxyethyl-2-bromoethyl hydrogenphosphate, and 2-(meth)acrylamideethyl dihydrogenphosphate.

Examples of other monofunctional polymerizable monomer having an acidic group include a monofunctional polymerizable monomer having a sulfo group in the molecule such as 2-(meth)aciylamide-2-methylpropanesulfonic acid and 10-sulfodecyl(meth)acrylate.

The amount of the polymerizable monomer (d-3) having an acidic group to be added is not particularly limited but it is preferable that 1 to 90 parts by mass of the polymerizable monomer (d-3) having an acidic group be contained in 100 parts by mass of the polymerizable monomer (D). When the amount of the polymerizable monomer (d-3) having an acidic group to be added is less than 1 part by mass, the acid etching effect or primer treatment effect may not be obtained. Therefore, the amount is more preferably at least 2 parts by mass and further preferably at least 5 parts by mass. On the other hand, when the amount of the polymerizable monomer (d-3) having an acidic group to be added exceeds 90 parts by mass, sufficiently high curability may not be obtained and therefore the bonding performance may be deteriorated. Accordingly, the amount is more preferably 80 parts by mass or less and further preferably 70 parts by mass or less.

### Crosslinkable polymerizable monomer (d-4)

Preferably, the polymerizable monomer (D) contains the crosslinkable polymerizable monomer (d-4) other than the polymerizable monomer (d-1). When a polymerizable composition containing the crosslinkable polymerizable monomer (d-4) is used as a dental composition, it has advantages such as a further improvement in bond strength.

The crosslinkable polymerizable monomers (d-4) can be used independently or two or more of them can be used in suitable combination. The crosslinkable polymerizable monomer (d-4) is not particularly limited. Examples thereof include an aromatic compound-based bifunctional polymerizable monomer, an aliphatic compound-based bifunctional polymerizable monomer, and trifunctional or higher polymerizable monomers.

Examples of the aromatic compound-based bifunctional polymerizable monomer include 2,2-bis((meth)acryloyloxy-phenyl)propane, 2,2-bis[4-(3-(meth)acryloyloxy)-2-hydroxypropoxyphenyl]propane (commonly known as "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)-propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromeritate.

Examples of the aliphatic compound-based bifunctional polymerizable monomer include ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, and 2,2,4-trimethylhexamethylene bis(2-carbamoyloxyethyl)dimethacrylate (commonly known as "UDMA").

Examples of the trifunctional or higher polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tetra(meth)acrylate, dipentaerythritol penta(meth)acrylate, dipentaerythritol hexa(meth)acrylate, N,N-(2,2,4-trimethylhexamethylene)bis[2-(aminocarboxy)propane-1,3-diol] tetramethacrylate, and 1,7-diacryloyloxy-2,2,6,6-tetraacryloyloxymethyl-4-oxyheptane.

The amount of the crosslinkable polymerizable monomer (d-4) to be added is not particularly limited, but it is preferable that 1 to 90 parts by mass of the crosslinkable polymerizable monomer (d-4) be contained in 100 parts by mass of the polymerizable monomer (D). When the amount of the crosslinkable polymerizable monomer (d-4) to be added is less than 1 part by mass, sufficiently high bond strength may not be obtained. Therefore, the amount is more preferably at least 2 parts by mass and further preferably at least 5 parts by mass. On the other hand, when the amount of the crosslinkable polymerizable monomer (d-4) to be added exceeds 90 parts by mass, the polymerizable composition may not penetrate sufficiently into a collagen layer of dentin and thereby high bond strength may not be obtained. Therefore, the amount is more preferably 80 parts by mass or less and further preferably 70 parts by mass or less.

In the polymerizable composition of the present invention, a weight ratio (D):(C) between the polymerizable monomer (D) and the tertiary amine (C) is 100:0.5 to 100:10. When the content of the tertiary amine (C) is low and out of this range, the acidity of the composition is increased and the hydrolysis of the polymerizable monomer (D) is accelerated, thereby lowering the storage stability (adhesive properties after long-term storage) of the composition. When the content of the tertiary amine (C) is high and out of this range, sufficient curability cannot be obtained, thereby deteriorating the adhesive properties. Preferably, the weight ratio (C):(D) is 100:0.5 to 100:5.

Preferably, in the polymerizable composition of the present invention, a total content of the bisacylphosphine oxide (A) and the α-diketone (B) is 0.1 to 20 parts by weight, more preferably 0.2 to 15 parts by weight, and further preferably 0.3 to 10 parts by weight, with respect to 100 parts by weight of the polymerizable monomer (D).

The polymerizable composition of the present invention may contain components other than the above-mentioned components depending on the application. The polymerizable composition of the present invention can be used in various applications that require photocurability, and it is particularly suitable for dental applications.

Examples of the components particularly useful for dental applications other than the above-mentioned components include a solvent (E) and a filler (F).

### Solvent (E)

The polymerizable composition of the present invention may contain the solvent (E) depending on the specific embodiment. Examples of the solvent include water (J), an organic solvent (K), and a mixed solvent thereof.

When the polymerizable composition of the present invention contains water (J), it exhibits both excellent bond strength and excellent bond durability. Preferably, the content of water (J) is 6 to 2000 parts by mass with respect to 100 parts by mass of the polymerizable monomer (D). When the content of water (J) is less than 6 parts by mass, the monomer may not have sufficiently high penetrability into a collagen layer and the bond strength may be reduced. On the other hand, when the content of water (F) exceeds 2000 parts by mass, the polymerizability of the monomer may be deteriorated and both the bond strength and bond durability may be reduced. The content of water (J) is more preferably at least 7 parts by mass and further preferably at least 10 parts by mass. Furthermore, the content of water (J) is more preferably 1500 parts by mass or less. Preferably, water (J) is free of impurities that have adverse effects, and distilled water or ion exchanged water is preferable.

The organic solvents (K) can be used independently or two or more of them can be used in suitable combination. Examples of the organic solvent (K) include methanol, ethanol, 1-propanol, 2-propanol, 1-butanol, 2-methyl-2-propanol, acetone, methyl ethyl ketone, tetrahydrofuran, diethyl ether, diisopropyl ether, hexane, toluene, chloroform, ethyl acetate, and butyl acetate. Particularly, when both safety to biological bodies and easy removal based on volatility are taken into consideration, the organic solvent (K) is preferably a water-soluble organic solvent. Specifically, ethanol, 2-propanol, 2-methyl-2-propanol, acetone, and tetrahydrofuran can be used preferably. The content of the organic solvent (K) is not particularly limited and the organic solvent (K) may not need to be added depending on the embodiment. In an embodiment using the organic solvent, it is preferable that 1 to 2000 parts by mass of the organic solvent (K) be contained with respect to 100 parts by mass of the polymerizable monomer (D). The preferable amount of the organic solvent (K) to be added varies considerably depending on the embodiment in which it is used. Therefore, preferable amounts of the organic solvents (K) to be added according to respective embodiments are indicated together with description of specific embodiments of the polymerizable composition of the present invention described later.

### Filler (F)

Preferably, the filler (F) further may be mixed into the polymerizable composition of the present invention depending on the embodiment. Generally, such fillers are divided roughly into organic fillers, inorganic fillers, and organic-inorganic composite fillers. Examples of materials for the organic fillers include polymethylmethacrylate, polyethylmethacrylate, a methylmethacrylate-ethylmethacrylate copolymer, cross-linked polymethylmethacrylate, cross-linked polyethylmethacrylate, polyamide, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used independently or a mixture of two or more of them may be used. The shapes of the organic fillers are not particularly limited, and particle sizes of the fillers to be used can be selected appropriately. From the viewpoints of, for example, handling ability and mechanical strength of the resultant polymerizable composition, the mean particle size of the organic fillers is preferably 0.001 to 50 µm and more preferably 0.001 to 10 µm,

Examples of materials for the inorganic fillers include quartz, silica, alumina, silica-titania, silica-titania-barium oxide, silica-zirconia, silica-alumina, lanthanum glass, borosilicate glass, soda glass, barium glass, strontium glass, glass ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. Similarly, these can be used independently or two or more of them can be used in mixture. The shapes of the inorganic fillers are not particularly limited and particle sizes of the fillers to be used can be selected appropriately. From the viewpoints of, for example, handling ability and mechanical strength of the resultant polymerizable composition, the mean particle size of the inorganic fillers is preferably 0.001 to 50 µm and more preferably 0.001 to 10 µm.

Examples of the shapes of the inorganic fillers include amorphous fillers and spherical fillers. From the viewpoint of improving the mechanical strength of the polymerizable composition, it is preferable that spherical fillers be used as the inorganic fillers. Furthermore, in the case of using the spherical fillers, when the polymerizable composition of the present invention is used as a dental composite resin, there also is an advantage that a composition resin with excellent surface smoothness is obtained. In this case, the spherical fillers are fillers in which when a photograph thereof is taken with a scanning electron microscope (hereinafter abbreviated as SEM), particles observed within a unit field of view are rounded and the mean uniformity obtained by dividing the particle size in the direction orthogonal to the maximum diameter by the maximum diameter is at least 0.6. The mean particle size of the spherical fillers is preferably 0.1 to 5 µm. When the mean particle size is less than 0.1 µm, the filling rate of the spherical fillers in the polymerizable composition decreases and thereby the mechanical strength may be reduced. On the other hand, when the mean particle size exceeds 5 µm, the surface areas of the spherical fillers are reduced and a cured body with high mechanical strength may not be obtained.

The inorganic fillers may be used after the surfaces thereof are treated beforehand with a known surface-treating agent such as a silane coupling agent in order to adjust fluidity of the polymerizable composition, as required. Examples of such a surface-treating agent include vinyltrimethoxysilane, vinyltriethoxysilane, vinyltrichlorosilane, vinyltri(β-methoxyethoxy)silane, γ-methacryloyloxypropyltrimethoxysilane, 11-methacryloyloxyundecyltrimethoxysilane, γ-glycidoxypropyltrimethoxysilane. γ-mercaptopropyltrimethoxysilane, and γ-aminopropyltriethoxysilane.

The organic-inorganic composite fillers can be obtained as follows. That is, a monomer compound is added to the aforementioned inorganic filler beforehand, this is made into a paste and is then polymerized, and thereafter this is crushed. The organic-inorganic composite filler that can be used is, for example, a TMPT filler (obtained by mixing trimethylolpropane methacrylate with a silica filler, polymerizing it, and then crushing it). The shape of the organic-inorganic composite filler is not particularly limited, and the particle size of the filler to be used can be selected appropriately. From the viewpoints of, for example, handling ability and mechanical strength of the resultant polymerizable composition, the mean particle size of the organic-inorganic composite filler is preferably 0.001 to 50 µm and more preferably 0.001 to 10 µm.

The amount of the filler (F) to be added is not particularly limited, but it is preferable that 1 to 2000 parts by mass of the filler (F) be contained with respect to 100 parts by mass of the polymerizable monomer (D). The preferable amount of the filler (F) to be added varies considerably depending on the embodiment to be employed. Accordingly, preferable amounts of the filler (F) to be added according to the respective embodiments are indicated together with description of specific embodiments of the polymerizable composition of the present invention described later.

The polymerizable composition of the present invention may contain a chemical polymerization initiator (G) and/or a polymerization accelerator (H) as long as they do not impair the advantageous effects of the present invention.

### Chemical polymerization initiator (G)

When the polymerizable composition of the present invention contains the chemical polymerization initiator (G), a dual curing type dental material can be obtained. The chemical polymerization initiator (G) is not particularly limited as long as it has no adverse effects on the photopolymerization initiation ability of the photopolymerization initiator composition (I), and an organic peroxide is preferably used. The organic peroxide used as the chemical polymerization initiator is not particularly limited and a known one can be used. Examples of typical organic peroxides include ketone peroxide, hydroperoxide, diacyl peroxide, dialkyl peroxide, peroxyketal, peroxyester, and peroxydicarbonate.

Examples of the ketone peroxide used as the chemical polymerization initiator include methyl ethyl ketone peroxide, methyl isobutyl ketone peroxide, methylcyclohexanone peroxide, and cyclohexanone peroxide.

Examples of the hydroperoxide used as the chemical polymerization initiator include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxide used as the chemical polymerization initiator include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxide used as the chemical polymerization initiator include di-t-butyl peroxide, dicumyl peroxide, t-butylcumyl peroxide, 2,5-dimethyl-2,5-di(t-butylperoxy)hexane, 1,3'bis(t-butylperoxyisopropyl)benzene, and 2,5-dimethyl-2,5-di(t-butylperoxy)-3-hexyne.

Examples of the peroxyketal used as the chemical polymerization initiator include 1,1-bis(t-butylperoxy)-3,3,5-trimethylcyclohelane, 1,1-bis(t-butylperoxy)cyclohexane, 2,2-bis(t-butylperoxy)butane, 2,2-bis(t-butylperoxy)octane, and 4,4-bis(t-butylperoxy)valeric acid-n-butyl ester.

Examples of the peroxyester used as the chemical polymerization initiator include α-cumyl peroxyneodecanoate, t-butyl peroxyneodecanoate, t-butyl peroxypivarate, 2,2,4-trimethylpentylperoxy-2-ethyl hexanoate, t-amylperoxy-2'ethyl hexanoate, t-butylperoxy-2-ethyl hexanoate, di-t-butylperoxy isophthalate, di-t-butylperoxy hexahydroterephthalate, t-butylperoxy-3,3,5-trimethyl hexanoate, t-butylperoxy acetate, t-butylperoxy benzoate, and t-butylperoxymaleic acid.

Examples of the peroxydicarbonate used as the chemical polymerization initiator include di-3-methoxy peroxydicarbonate, di-2-ethylhexyl peroxydicarbonate, bis(4-t-butylcyclohexyl)peroxydicarbonate, diisopropyl peroxydicarbonate, di-n-propyl peroxydicarbonate, di-2-ethoxyethyl peroxydicarbonate, and diallyl peroxydicarbonate.

Among these organic peroxides, diacyl peroxides are used preferably from the viewpoint of a comprehensive balance of safety, storage stability, and radical production ability, and among these, benzoyl peroxide is used particularly preferably.

### Polymerization accelerator (H)

Examples of the polymerization accelerator (H) include primary or secondary amines, sulfinic acids and salts thereof, borate compounds, barbituric acid derivatives, triazine compounds, copper compounds, tin compounds, vanadium compounds, halogen compounds, aldehydes, thiol compounds, sulfite, bisulfite, and thiourea compounds.

Examples of the primary or secondary amines include: primary amines such as n-butylamine, n-hexylamine, and n-octylamine; and secondary amines such as diisopropylamine, dibutylamine, and N-methylethanolamine.

Examples of sulfinic acid and salt thereof used as the polymerization accelerator (H) include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Sodium benzenesulfinate, sodium p-toluenesulfinate, and sodium 2,4,6-triisopropylbenzenesulfinate are particularly preferable.

The borate compound used as the polymerization accelerator (H) is preferably an arylborate compound. Specific examples of the arylborate compound used preferably include, as a borate compound having one aryl group in one molecule, a sodium salt, lithium salt, potassium salt, magnesium salt, tetrabutylammonium salt, tetramethylammonium salt, tetraethylammonium salt, methylpyridinium salt, ethylpyridinium salt, butylpyridinium salt, methylquinolinium salt, ethylquinolinium salt, and butylquinolinium salt of trialkylphenylboron, trialkyl(p-chlorophenyl)-boron, trialkyl(p-fluorophenyl)boron, trialkyl(3,5-bistrifluoromethyl)phenylboron, trialkyl[3,5-bis(1,1,1,3,3,3-hexafluoro-2-methoxy-2-propyl)phenyl]boron, trialkyl(p-nitrophenyl)boron, trialkyl(m-nitrophenyl)boron, trialkyl(p-butylphenyl)boron, trialkyl(m-butylphenyl)boron, trialkyl(p-butyloxyphenyl)boron, trialkyl(m-butyloxyphenyl)boron, trialkyl(p-octyloxyphenyl)boron, and trialkyl(m-octyloxyphenyl)boron (each alkyl group is at least one selected from the group consisting of, for example, an n-butyl group, an n-octyl group, and an n-dodecyl group).

Examples of the borate compound having two aryl groups in one molecule include a sodium salt, lithium salt, potassium salt, magnesium salt, tetrabutylammonium salt, tetramethylammonium salt, tetraethylammonium salt, methylpyridinium salt, ethylpyridinium salt, butylpyridinium salt, methylquinolinium salt, ethylquinolinium salt, and butylquinolinium salt of dialkyldiphenylboron, dialkyldi(p-chlorophenyl)boron, dialkyldi(p-fluorophenyl)boron, dialkyldi(3,5-bistrifluoromethyl)phenylboron dialkyldi[3,5-bis(1,1,1,3,3,3-hexafluoro-2-methoxy-2-propyl)phenyl]boron, dialkyldi(p-nitrophenyl)boron, dialkyldi(m-nitrophenyl)boron, dialkyldi(p-butylphenyl)boron, dialkyldi(m-butylphenyl)boron, dialkyldi(p-butyloxyphenyl)boron, dialkyldi(m-butyloxyphenyl)boron, dialkyldi(p-octyloxyphenyl)boron, and dialkyldi(m-octyloxyphenyl)boron (each alkyl group is at least one selected from the group consisting of, for example, an n-butyl group, an n-octyl group, and an n-dodecyl group).

Furthermore, examples of the borate compound having three aryl groups in one molecule include a sodium salt, lithium salt, potassium salt, magnesium salt, tetrabutylammonium salt, tetramethylammonium salt, tetraethylammonium salt, methylpyridinium salt, ethylpyridinium salt, butylpyridinium salt, methylquinolinium salt, ethylquinolinium salt, and butylquinolinium salt of monoalkyltriphenylboron, monoalkyltri(p-chlorophenyl)boron, monoalkyltri(p-fluorophenyl)boron, monoalkyltri(3,5-bistrifluoromethyl)phenylboron, monoalkyltri[3,5-bis(1,1,1,3,3,3-hexafluoro-2-metholy-2-propyl)phenyl]boron, monoalkyltri(p-nitrophenyl)boron; monoalkyltri(m-nitrophenyl)boron, monoalkyltri(p-butylphenyl)boron, monoalkyltri(m-butylphenyl)boron, monoalkyltri(p-butyloxyphenyl)boron, monoalkyltri(m-butyloxyphenyl)boron, monoalkyltri(p-octyloxyphenyl)boron, and monoalkyltri(m-octyloxyphenyl)boron (each alkyl group is one selected from, for example, an n-butyl group, an n-octyl group, and an n-dodecyl group).

Furthermore, examples of the borate compound having four aryl groups in one molecule include a sodium salt, lithium salt, potassium salt, magnesium salt, tetrabutylammonium salt, tetramethylammonium salt, tetraethylammonium salt, methylpyridinium salt, ethylpyridinium salt, butylpyridinium salt, methylquinolinium salt, ethylquinolinium salt, and butylquinolinium salt of tetraphenylboron, tetrakis(p-chlorophenyl)boron, tetrakis(p-fluorophenyl)boron, tetrakis(3,5-bistrifluoromethyl)phenylboron, tetrakis[3,5-bis(1,1,1,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, (3,5-bistrifluoromethyl)phenyltriphenylboron, (p-nitrophenyl)triphenylboron, (m-butyloxyphenyl)triphenylboron, (p-butyloxyphenyl)triphenylboron, (m-octyloxyphenyl)triphenylboron, and (p-octyloxyphenyl)triphenylboron.

More preferably, from the viewpoint of storage stability, among these arylborate compounds, a borate compound having three or four aryl groups in one molecule is used. Furthermore, one of these arylborate compounds can be used or two or more of them can be used in mixture.

Examples of the barbituric acid derivative used as the polymerization accelerator (H) include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-n-butylbarbituric acid, 1,3-dimethyl-5-isobutylbarbituric acid, 1,3-dimethylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, and thiobarbituric acids, as well as salts thereof (particularly, alkali metals or alkaline earth metals are preferable). Examples of salts of these barbituric acids include sodium 5-butylbarbiturate, sodium 1,3,5-trimethylbarbiturate, and sodium 1-cyclohexyl-5-ethylbarbiturate.

Examples of particularly preferable barbituric acid derivatives include 5-butylbarbituric acid, 1,3,5-trimethylbarbituric acid, 1-cyclohexyl-5-ethylbarbituric acid, 1-benzyl-5-phenylbarbituric acid, and sodium salts of these barbituric acids.

Examples of the triazine compound used as the polymerization accelerator (H) include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,β-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimetholyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N,N-bis(2-hydroxyethyl)amino}ethoxy]-4,6-bis(trichloromethyl)-s-triazine, 2-[2-{N-hydroxyethyl-N-ethylamino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N,N-diallylamino}ethoxy]-4,6-bis(trichloromethyl)-s-triazine.

Particularly preferable ones among the triazine compounds described above as examples are 2,4,6-tris(trichloromethyl)-s-triazine in terms of polymerization activity and 2-phenyl-4,6-bis(trichloromethyl)-s-triazine, 2-(p-chlorophenyl)-4,6-bis(trichloromethyl)-s-triazine, and 2-(4-biphenylyl)-4,6-bis(trichloromethyl)-s-triazine in terms of storage stability. One of the above-mentioned triazine compounds may be used or two or more of them may be used in mixture.

Examples of the copper compound used preferably as the polymerization accelerator (H) include copper acetylacetonate, copper (II) acetate, copper oleate, copper (II) chloride, and copper (II) bromide.

Examples of the tin compound used as the polymerization accelerator (H) include di-n-butyltin dimalate, di-n-octyltin dimalate, di-n-octyltin dilaurate, and di-n-butyltin dilaurate. Particularly preferable tin compounds are di-n-octyltin dilaurate and di-n-butyltin dilaurate.

The vanadium compound used as the polymerization accelerator (H) is preferably one of tetravalent and/or pentavalent vanadium compounds. Examples of the tetravalent and/or pentavalent vanadium compounds include compounds described in JP 2003-96122 A such as divanadium (IV) tetroxide, vanadyl (IV) acetylacetonate, vanadyl (IV) oxalate, vanadyl (IV) sulfate, oxobis(1-phenyl-1,3-butanedionate)vanadium (IV), bis(maltolato)oxovanadium (IV), vanadium (V) pentoxide, sodium metavanadate (V), and ammonium metavanadate (V).

Examples of the halogen compound used preferably as the polymerization accelerator (H) include dilauryldimethylammoniumchloride, lauryldimethylbenzylammoniumchloride, benzyltrimethylammoniumchloride, tetramethylammoniumchloride, benzyldimethylcetylammoniumchloride, and dilauryldimethylammoniumbromide.

Examples of the aldehydes used as the polymerization accelerator (H) include terephthalaldehyde and a benzaldehyde derivative. Examples of the benzaldehyde derivative include dimethylaminobenzaldehyde, p-methyloxybenzaldehyde, p-ethyloxybenzaldehyde, and p-n-octyloxybenzaldehyde. Among these, from the viewpoint of curability, p-n-octyloxybenzaldehyde is used preferably.

Examples of the thiol compound used as the polymerization accelerator (H) include 3-mercaptopropyltrimethoxysilane, 2-mercaptobenzooxazol, decanethiol, and thiobenzoic acid.

Examples of the sulfite used as the polymerization accelerator (H) include sodium sulfite, potassium sulfite, calcium sulfite, and ammonium sulfite.

Examples of the bisulfate used as the polymerization accelerator (H) include sodium bisulfate and potassium bisulfate.

Examples of the thiourea compound used as the polymerization accelerator (H) include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea tricyclohexylthiourea, tetramethylthiourea, tetraethylthiourea, tetra-n-propylthiourea, and tetracyclohexylthiourea.

The polymerizable composition of the present invention may contain a fluoride as long as it does not impair the advantageous effects of the present invention. The fluoride is not particularly limited as long as it can be dissolved in water and release a fluorine ion. Specific examples thereof include lithium fluoride, sodium fluoride, potassium fluoride, rubidium fluoride, cesium fluoride, beryllium fluoride, magnesium fluoride, calcium fluoride, strontium fluoride, barium fluoride, aluminum fluoride, manganese (II) fluoride, Iron (II) fluoride, Iron (III) fluoride, cobalt (II) fluoride, copper (II) fluoride, zinc fluoride, antimony (III) fluoride, lead (II) fluoride, silver (I) fluoride, cadmium fluoride, tin (II) fluoride, tin (IV) fluoride, diamine silver fluoride, ammonium fluoride, sodium hydrogenfluoride, ammonium hydrogendifluoride, potassium hydrogenfluoride, sodium fluorophosphate, potassium hexafluorotitanate, sodium hexafluorosilicate, sodium hexafluorophosphate, sodium hexafluorotin (IV), alanine hexafluorostannate (IV), sodium pentafluorostannate (II), and potassium hexafluorozirconate.

In addition, for example, a pH adjuster, polymerization inhibitor, ultraviolet absorbent, thickening agent, colorant, antibacterial agent, and flavor may be added to the polymerizable composition of the present invention as long as they do not impair the advantageous effects of the present invention.

In dental applications, the polymerizable composition of the present invention can be used specifically for dental materials such as a primer, bonding material, composite resin, cement (resin cement, glass ionomer cement, and resin-reinforced glass ionomer cement), pit and fissure sealant, and denture base resin. Particularly, it can be used suitably as a primer, bonding material, composite resin, or cement, among which it can be used as the bonding material more suitably. In this case, the polymerizable composition may be used as a two component type in which the components of the composition are divided into two.

The dental materials can be obtained by selecting the polymerizable monomer (D) according to a composition of a known photocurable dental material, and adding, if needed, the solvent (E), the filler (F), etc. thereinto.

Since the polymerizable monomer (d-1) includes new polymerizable monomers and the polymerizable monomer (D) contains preferably the polymerizable monomer (d-1), hereinafter will be described examples in which dental materials (a primer, a bonding material, a composite resin, and a cement) are produced using the polymerizable monomer (d-1).

### Dental primer

Recently, in order to simplify the operation process, a product that allows the decalcifying step and the penetration step to be performed together in one step has been developed and has been used practically. The product is referred to as a "self-etching primer". Generally, the bonding system using a self-etching primer and a bonding material is referred to as a "two-step bonding system". The polymerizable monomer (d-1) has at least two hydroxyl groups and has high hydrophilicity. Therefore, it easily penetrates into a collagen layer of dentin. Accordingly, the polymerizable composition containing the polymerizable monomer (d-1) can be used as a dental primer, and also can be used as a dental self-etching primer.

Preferably, the primer using the polymerizable composition of the present invention is a composition containing the polymerizable monomer (d-1), the photopolymerization initiator composition (I), the polymerizable monomer (d-3) having an acidic group, and the solvent (E). The amounts of the (d-1) and (d-3) to be added are preferably 10 to 99 parts by mass of (d-1) and 1 to 90 parts by mass of (d-3), more preferably 15 to 98 parts by mass of (d-1) and 2 to 85 parts by mass of (d-3), and further preferably 20 to 97 parts by mass of (d-1) and 3 to 80 parts by mass of (d-3), in 100 parts by mass of the polymerizable monomer (D).

Furthermore, when penetrability of a primer composition into a tooth structure (particularly dentin) is considered important, it is preferable that the polymerizable monomer (d-2) having one polymerizable group and at least one hydroxyl group be contained furhter. When the primer composition contains (d-1), (d-2), and (d-3), the amounts of respective components to be added are preferably 1 to 98 parts by mass of (d-1), 1 to 90 parts by mass of (d-2), and 1 to 90 parts by mass of (d-3), more preferably 3 to 90 parts by mass of (d-1), 5 to 85 parts by mass of (d-2), and 2 to 80 parts by mass of (d-3), and further preferably 10 to 80 parts by mass of (d-1), 7 to 80 parts by mass of (d-2), and 3 to 60 parts by mass of (d-3), in 100 parts by mass of the polymerizable monomer (D).

When particularly the strength of the cured product of a primer using the polymerizable composition of the present invention is intended to be improved, the crosslinkable polymerizable monomer (d-4) further may be added. When consideration is given to penetrability into a tooth structure (particularly dentin), the above-mentioned (d-4) is preferably an aliphatic compound-based bifunctional polymerizable monomer, more preferably ethylene glycol di(meth)acrylate, diethylene glycol di(meth)acrylate, triethylene glycol di(meth)acrylate, or 1,2-bis(3-methacryloyloxy-2-hydroxypropoxy)ethane, and further preferably triethylene glycol di(meth)acrylate, or 1,2-bis(3-methacryloyloxy-2-hydroxypropoxy)ethane. The amount of the above-mentioned (d-4) to be added is preferably 0 to 30 parts by mass, more preferably 1 to 25 parts by mass, and further preferably 3 to 20 parts by mass, in 100 parts by mass of the polymerizable monomer (D).

The weight ratio (D):(C) between the polymerizable monomer (D) and the tertiary amine (C) is 100:0.5 to 100:10, and preferably 100:0.5 to 100:5. As for the amount of the photopolymerization initiator composition (I) to be added, the total content of the bisacylphosphine oxide (A) and the α-diketone (B) in the photopolymerization initiator composition (I) is preferably 0.1 to 20 parts by weight, more preferably 0.2 to 10 parts by weight, and further preferably 0.3 to 8 parts by weight, with respect to 100 parts by weight of the polymerizable monomer (D).

When the polymerizable composition of the present invention containing the polymerizable monomer (d-1) is used as a primer, the polymerizable composition of the present invention can have higher hydrophilicity and improved penetrability into a collagen layer of dentin because the polymerizable monomer (d-1) has at least two hydroxyl groups in a molecule. Furthermore, it is preferable that the solvent (E) be used in the form of a mixed solvent of water (J) and the organic solvent (K). The amount of water (J) to be contained in the mixed solvent is not particularly limited but is preferably at least 10 mass%, more preferably at least 30 mass%, and further preferably at least 50 mass%. Moreover, the organic solvent (K) may not need to be added depending on the embodiment. The amount of the aforementioned solvent (E) to be added is preferably 5 to 4000 parts by mass, more preferably 10 to 3000 parts by mass, and further preferably 15 to 2000 parts by mass, with respect to 100 parts by mass of the polymerizable monomer (D). Furthermore, when the solvent (E) is used in the form of a mixed solvent of water (J) and the organic solvent (K), the amounts of the aforementioned (J) and (K) to be added are preferably 4 to 2000 parts by mass of (J) and 1 to 2000 parts by mass of (K), more preferably 8 to 1500 parts by mass of (J) and 2 to 1500 parts by mass of (K), and further preferably 12 to 1000 parts by mass of (J) and 3 to 1000 parts by mass of (K), with respect to 100 parts by mass of the polymerizable monomer (D).

### Dental bonding material

The polymerizable composition of the present invention is used preferably as a bonding material. Preferably, the bonding material in the aforementioned "two-step bonding system" is a composition containing the polymerizable monomer (d-1), the photopolymerization initiator composition (I), and the filler (F). More preferably, such a composition further contains the polymerizable monomer (d-2) having one polymerizable group and at least one hydroxyl group and/or the crosslinkable polymerizable monomer (d-4). The amounts of the respective components to be added are preferably 1 to 99 parts by mass of (d-1), 0 to 90 parts by mass of (d-2), and 0 to 90 parts by mass of (d-4) and more preferably 2 to 96 parts by mass of (d-1), 1 to 80 parts by mass of (d-2), and 1 to 80 parts by mass of (d-4), in 100 parts by mass of the polymerizable monomer (D). As in the case of the above-mentioned (d-1), the use of a compound having at least two polymerizable groups allows a cured product to have increased mechanical strength. From such a viewpoint, the aforementioned (d-4) is more preferably a polymerizable monomer having at least two polymerizable groups, and from a viewpoint of obtaining a cured product with particularly high strength, it is further preferable that the aforementioned (d-4) contain an aromatic compound-based bifunctional polymerizable monomer. The use of an aliphatic bifunctional polymerizable monomer and aromatic compound-based bifunctional polymerizable monomer in combination as the aforementioned (d-4) also is a preferable embodiment.

The weight ratio (D):(C) between the polymerizable monomer (D) and the tertiary amine (C) is 100:0.5 to 100:10, and preferably 100:0.5 to 100:5. As for the amount of the photopolymerization initiator composition (I) to be added, the total content of the bisacylphosphine oxide (A) and the α-diketone (B) in the photopolymerization initiator composition (I) is preferably 0.1 to 20 parts by weight, more preferably 0.2 to 15 parts by weight, and further preferably 0.3 to 10 parts by weight, with respect to 100 parts by weight of the polymerizable monomer (D). Moreover, the amount of the filler (F) to be added is preferably 1 to 20 parts by mass, more preferably 2 to 17 parts by mass, and further preferably 3 to 15 parts by mass, with respect to 100 parts by mass of the polymerizable monomer (D).

Recently, since there are demands for further simplification in operations, products that allow three steps of "decalcifying", "penetration", and "curing" to be performed together in one step also have been developed and are referred to as "one-step bonding systems". Two typical products of the bonding material used in such a one-step bonding system are a bonding material in which two separate liquids of liquid A and liquid B are mixed together immediately before use and a bonding material that is provided in the form of one liquid from the beginning and that is a so-called one-component one-step bonding system. Among these, the one-component type product further simplifies the process and therefore has a greater advantage in use. When the polymerizable composition of the present invention is used as the bonding material of the aforementioned one-component one-step bonding system, the composition is preferably a composition containing the polymerizable monomer (d-1), the photopolymerization initiator composition (I), the polymerizable monomer (d-3) having an acidic group, the filler (F), and the solvent (E), and further preferably, such a composition further contains the crosslinkable polymerizable monomer (d-4). The amounts of respective components to be added are preferably 1 to 98 parts by mass of (d-1), 1 to 90 parts by mass (d-3), and 0 to 90 parts by mass of (d-4), more preferably 2 to 94 parts by mass of (d-1), 2 to 80 parts by mass of (d-3), and 2 to 80 parts by mass of (d-4), and further preferably 7 to 90 parts by mass of (d-1), 3 to 70 parts by mass of (d-3), and 7 to 70 parts by mass of (d-4), in 100 parts by mass of the polymerizable monomer (D). In the one-component one-step bonding system, since the "penetration" and "curing" are performed at one time, the use of a polymerizable monomer having at least two hydroxyl groups and at least two polymerizable groups like the aforementioned (d-1) is of great significance.

In the one-component one-step bonding system, when penetrability into a tooth structure (particularly dentin) is considered important, it is preferable that the polymerizable monomer (d-2) having one polymerizable group and at least one hydroxyl group further be contained. When the one-component one-step bonding system contains (d-1), (d-2), (d-3), and (d-4), the amounts of the respective components to be added are preferably 1 to 95 parts by mass of (d-1), 1 to 90 parts by mass of (d-2), 1 to 95 parts by mass of (d-3), and 3 to 97 parts by mass of (d-4), more preferably 3 to 90 parts by mass of (d-1), 3 to 85 parts by mass of (d-2), 2 to 80 parts by mass of (d-3), and 5 to 80 parts by mass of (d-4), and further preferably 5 to 80 parts by mass of (d-1), 5 to 80 parts by mass of (d-2), 3 to 60 parts by mass of (d-3), and 8 to 70 parts by mass of (d-4), in 100 parts by mass of the polymerizable monomer (D).

The weight ratio (D):(C) between the polymerizable monomer (D) and the tertiary amine (C) is 100:0.5 to 100:10, and preferably 100:0.5 to 100:5. As for the amount of the photopolymerization initiator composition (I) to be added, the total content of the bisacylphosphine oxide (A) and the α-diketone (B) in the photopolymerization initiator composition (I) is preferably 0.1 to 20 parts by weight, more preferably 0.2 to 15 parts by weight, and further preferably 0.5 to 10 parts by weight, with respect to 100 parts by weight of the polymerizable monomer (D). Furthermore, the amount of the filler (F) to be added is preferably 1 to 20 parts by mass, more preferably 1.5 to 15 parts by mass, and further preferably 2 to 10 parts by mass, with respect to 100 parts by mass of the polymerizable monomer (D).

The amount of the solvent (E) to be added is preferably 6 to 4000 parts by mass, more preferably 12 to 3000 parts by mass, and further preferably 15 to 2000 parts by mass, with respect to 100 parts by mass of the polymerizable monomer (D). The one-component one-step bonding system needs to perform all the processes of decalcifying, penetration, and curing with one liquid in one step. Therefore, from the viewpoint that penetrability is considered important, it is preferable that water (J) be contained as the solvent (E). On the other hand, from the viewpoint that curability is considered important, it is preferable that the bonding system contain a suitable amount of the crosslinkable polymerizable monomer (d-4). From the viewpoints of increasing the solubility of the aforementioned (d-4) and obtaining a uniform solution, it is preferable that the organic solvent (K) be contained as the aforementioned solvent (E). A more preferable embodiment is the use of the solvent (E) in the form of a mixed solvent of water (J) and the organic solvent (K). In such an embodiment, the amounts of the aforementioned (J) and (K) to be added are preferably 2 to 2000 parts by mass of (J) and 4 to 2000 parts by mass of (K), more preferably 4 to 1500 parts by mass of (J) and 8 to 1500 parts by mass of (K), and further preferably 5 to 1000 parts by mass of (J) and 10 to 1000 parts by mass of (K), with respect to 100 parts by mass of the polymerizable monomer (D).

### Dental composite resin

The polymerizable composition of the present invention is used preferably as composite resin. In this case, the polymerizable composition is preferably a composition containing the polymerizable monomer (d-1), the crosslinkable polymerizable monomer (d-4), the photopolymerization initiator composition (I), and the filler (F). Generally, the composite resin is used in the form of filling a cavity after the cavity is formed by cutting a site of caries incidence. Thereafter, generally, the composite resin filling the cavity is cured through photopolymerization. Therefore, the use of the photopolymerization initiator composition (I) has significance. Furthermore, since the composite resin that has filled the cavity and that has been cured as described above is subjected to occlusal pressure inside an oral cavity, high mechanical strength is required. Accordingly, the content of the filler (F) in the composition is preferably 30 to 2000 parts by mass, and more preferably 50 to 1500 parts by mass, with respect to 100 parts by mass of the polymerizable monomer (D). When the content of the filler (F) is less than 30 parts by mass, mechanical strength of the cured product may be insufficient. On the other hand, when the content of the filler (F) exceeds 2000 parts by mass, it may become difficult to disperse the filler (F) uniformly throughout the polymerizable monomer (D), which may result in a composition that is insufficient in mechanical strength and handleability. The amounts of the respective components to be added are preferably 1 to 99 parts by mass of (d-1) and 1 to 99 parts by mass of (d-4), more preferably 10 to 95 parts by mass of (d-1) and 5 to 90 parts by mass of (d-4), and further preferably 15 to 90 parts by mass of (d-1) and 10 to 85 parts by mass of (d-4), in 100 parts by mass of the polymerizable monomer (D). The weight ratio (D):(C) between the polymerizable monomer (D) and the tertiary amine (C) is 100:0.5 to 100:10, and preferably 100:0.5 to 100:5. As for the amount of the photopolymerization initiator composition (I) to be added, the total content of the bisacylphosphine oxide (A) and the α-diketone (B) in the photopolymerization initiator composition (I) is preferably 0.1 to 20 parts by weight, more preferably 0.2 to 10 parts by weight, and further preferably 0.3 to 5 parts by weight, with respect to 100 parts by weight of the polymerizable monomer (D).

Since the polymerizable monomer (d-1) has both excellent curability and penetrability into a tooth structure, it is preferable that the polymerizable composition be used particularly as a self-adhesive composite resin among composite resins. When the polymerizable composition of the present invention is used as a self-adhesive composite resin, it is preferable that the composition contain the polymerizable monomer (d-1), the polymerizable monomer (d-3) having an acidic group, the crosslinkable polymerizable monomer (d-4), the photopolymerization initiator composition (I), and the filler (F). The amounts of respective components to be added are preferably 1 to 95 parts by mass of (d-1), 1 to 90 parts by mass of (d-3), and 4 to 90 parts by mass of (d-4), more preferably 5 to 80 parts by mass of (d-1), 2 to 85 parts by mass of (d-3), and 6 to 85 parts by mass of (d-4), and further preferably 10 to 80 parts by mass of (d-1), 3 to 80 parts by mass of (d-3), and 8 to 80 parts by mass of (d-4), in 100 parts by mass of the polymerizable monomer (D). Furthermore, when the penetrability into a tooth structure (particularly dentin) is considered important, it is preferable that the composition further contain the polymerizable monomer (d-2) having one polymerizable group and at least one hydroxyl group. When the self-adhesive composite resin contains (d-1), (d-2), (d-3), and (d-4), the amounts of the respective components to be added are preferably 1 to 95 parts by mass of (d-1), 1 to 90 parts by mass of (d-2), 1 to 90 parts by mass of (d-3), and 3 to 90 parts by mass (d-4), more preferably 3 to 90 parts by mass of (d-1), 3 to 85 parts by mass of (d-2), 2 to 85 parts by mass of (d-3), and 5 to 85 parts by mass of (d-4), and further preferably 5 to 80 parts by mass of (d-1), 5 to 70 parts by mass of (d-2), 3 to 60 parts by mass of (d-3), and 8 to 70 parts by mass of (d-4), in 100 parts by mass of the polymerizable monomer (D). With respect to the amounts of the photopolymerization initiator composition (I) and the filler (F) to be added, the same amounts as those used in the aforementioned general composite resin can be employed. Moreover, when particularly the penetrability into a tooth structure is considered important, it also is possible to add the solvent (E), and it is further preferable that the solvent (E) contain water (J). The amount of the solvent (E) to be added is preferably 0 to 15 parts by mass, and more preferably 1 to 10 parts by mass, with respect to 100 parts by mass of the polymerizable monomer (D).

### Dental cement

The polymerizable composition of the present invention can be used also as a dental cement. Examples of preferable cements include a resin cement, glass ionomer cement, and resin-reinforced glass ionomer cement. When the polymerizable composition of the present invention is used as a resin cement, the composition is preferably a composition containing the polymerizable monomer (d-1), the crosslinkable polymerizable monomer (d-4), the photopolymerization initiator composition (I), the polymerization accelerator (H), the filler (F), and water (J) to serve as the solvent (E). Such a composition further can contain the polymerizable monomer (d-3) having an acidic group. The dental cement is used suitably as, for example, a luting material that is used in fixing a metal or ceramics dental crown restorative material, which is referred to as an "inlay" or "crown", to a tooth. As in the case of the aforementioned (d-1), when at least two polymerizable groups are included, the resultant cured product has increased mechanical strength and can withstand, for example, occlusal pressure. From such a viewpoint, it is more preferable that the aforementioned (d-4) be a polymerizable monomer having at least two polymerizable groups. Furthermore, in the case of the form of usage as described above, since many of the dental crown restorative materials have optical opacity, it is not easy to cure the cement by only photopolymerization. Therefore, it is preferable that a chemical polymerization initiator additionally be used as the polymerization initiator (G). Furthermore, when polymerization is performed by using a chemical polymerization initiator, it is preferable to increase the amount of the tertiary amine (C) to be added to the photopolymerization initiator composition (I), or to use sulfinic acid and a salt thereof as the polymerization accelerator (H), in order to improve the reactivity of the chemical polymerization initiator. The filler (F) used is not particularly limited.

When the cement is intended to be provided with a property of sustained-release of fluoride, it is preferable that at least one selected from the group consisting of fluoroaluminosilicate glass, calcium fluoroaluminosilicate glass, strontium fluoroaluminosilicate glass, barium fluoroaluminosilicate glass, and strontium calcium fluoroaluminosilicate glass be used as the filler (F), and it is more preferable that fluoroaluminosilicate glass and/or barium fluoroaluminosilicate glass be used as the filler (F). On the other hand, when the cement is intended to be provided with radiopacity, it is preferable that at least one selected from the group consisting of barium glass, strontium glass, barium boroaluminosilicate glass, strontium boroaluminosilicate glass, strontium fluoroaluminosilicate glass, and barium fluoroaluminosilicate glass be used as the filler (F), and it is more preferable that barium glass and/or barium fluoroaluminosilicate glass be used as the filler (F).

When a chemical polymerization initiator is used, from the viewpoint of storage stability, it is preferable that the aforementioned (G) and (H) be stored in separate containers, respectively. That is, in a preferred embodiment, the resin cement is used in the form of a two component type. In a more preferred embodiment, the resin cement is used in the form of two paste type. Preferably, the respective pastes are stored while being separated from each other, the two pastes are mixed together immediately before use, and thereby chemical polymerization is allowed to proceed to cure the mixture. The aforementioned pastes each are prepared by mixing a liquid component of, for example, polymerizable monomer with the filler (F) (powder) together. Furthermore, when sulfinic acid and a salt thereof are used as the aforementioned (H), from the viewpoint of storage stability, it is preferable that the aforementioned (d-3) and (H) be stored in separate containers, respectively. Suppose that the aforementioned two pastes are referred to as a paste A and a paste B, respectively, an embodiment in which the paste A contains (d-1), (d-3), (I), (G), and (F) and the paste B contains (d-1), (H), and (F) is used particularly suitably.

When the polymerizable composition of the present invention is used as a dental cement, the amounts of the respective components to be added are not particularly limited. However, in 100 parts by mass of the polymerizable monomer (D), the composition contains preferably 1 to 98 parts by mass of (d-1), 0 to 90 parts by mass of (d-2), 1 to 90 parts by mass of (d-3), and 1 to 90 parts by mass of (d-4), and more preferably 2 to 96 parts by mass of (d-1), 0 to 85 parts by mass of (d-2), 2 to 85 parts by mass of (d-3), and 2 to 85 parts by mass of (d-4). The weight ratio (D):(C) between the polymerizable monomer (D) and the tertiary amine (C) is 100:0.5 to 100:10, and preferably 100:0.5 to 100:5. As for the amounts of the aforementioned (I), (G), and (H) to be added, when consideration is given to obtaining a suitable curing time, it is preferable that the total content of (A) and (B) in (I) be 0.01 to 20 parts by mass, the content of (G) be 0.001 to 20 parts by mass, and the content of (H) be 0.001 to 30 parts by mass, and it is more preferable that the total content of (A) and (B) in (I) be 0.1 to 20 parts by mass, the content of (G) be 0.05 to 15 parts by mass, and the content of (H) be 0.05 to 20 parts by mass, with respect to 100 parts by mass of the polymerizable monomer (D).

Moreover, the content of (F) is preferably 30 to 2000 parts by mass and more preferably 50 to 1500 parts by mass, with respect to 100 parts by mass of the polymerizable monomer (D). When the content of (F) is less than 30 parts by mass, mechanical strength of the cured product may be insufficient. On the other hand, in the case where the content of (F) exceeds 2000 parts by mass, when the resin cement is used as a two-paste-type cement, which is a preferred embodiment, the pastes lack fluidity, which makes it difficult to carry out sufficient mixing, and therefore the cured product may have reduced strength.

The polymerizable composition of the present invention is used preferably as a glass ionomer cement and more preferably as a resin-reinforced glass ionomer cement. The glass ionomer cement is typically one in which an inorganic filler such as fluoroaluminosilicate glass and polyalkenoic acid such as polyacrylic acid are reacted with each other through an acid-base reaction to be cured. Conceivably, the polyacrylic acid interacts with calcium contained in hydroxyapatite composing a tooth structure and thereby a bonding function is exhibited. When the polymerizable composition of the present invention is used as a glass ionomer cement, particularly preferably as a resin-reinforced glass ionomer cement, the polymerizable composition is preferably a composition containing (d-1), (I), (H), (F), (E), and polyalkenoic acid, and more preferably a composition containing (d-1), (d-4), (I), (H), (F), (E), and polyalkenoic acid, a composition containing (d-1), (d-2), (I), (H), (F), (E), and polyalkenoic acid, or a composition containing (d-1), (d-2), (d-4), (I), (H), (F), (E), and polyalkenoic acid. Such compositions further can contain (d-3).

The polymerizable monomer (d-2) having one polymerizable group and at least one hydroxyl group to be used is not particularly limited. As described later, from the viewpoint that an acid-base reaction is allowed to proceed smoothly, it is preferable that the solvent (E) contain water (J). Therefore, from the viewpoints of maintaining the uniformity of the polymerizable composition and obtaining consistent performance, it is more preferable that a monomer with high affinity for water (J) be used as the aforementioned (d-2). Such a monomer with high affinity for water (J) is preferably 2-hydroxyethyl(meth)acrylate, 3-hydroxypropyl(meth)acrylate, glycerol mono(meth)acrylate, or erythritol mono(meth)acrylate, and particularly preferably 2-hydroxyethylmethacrylate.

The crosslinkable polymerizable monomer (d-4) to be used is not particularly limited, but as described above, the use of a monomer with high affinity for water (J) as the aforementioned (d-4) is more preferable from the viewpoints of maintaining the uniformity of the polymerizable composition and obtaining consistent performance. In terms of a balance between such affinity for water (J) and the mechanical strength of the cured product, the aforementioned (d-4) is preferably an aliphatic compound-based bifunctional polymerizable monomer, and more preferably triethylene glycol di(meth)acrylate, polyethylene glycol di(meth)acrylate, neopentyl glycol di(meth)acrylate, 1,2-bis(3-methacryloyloxy-2-hydroxypropoxy)ethane, or 2,2,4-trimethylhexamethylenebis(2-carbamoyloxyethyl) dimethacrylate (commonly known as "UDMA").

The aforementioned polyalkenoic acid is a polymer of unsaturated monocarboxylic acid or unsaturated dicarboxylic acid. Specific examples of the polyalkenoic acid include homopolymers of, for example, acrylic acid, methacrylic acid, 2-chloroacrylic acid, 2-cyanoacrylic acid, aconitic acid, mesaconic acid, maleic acid, itaconic acid, fumaric acid, glutaconic acid, citraconic acid, and utraconic acid, or copolymers of these unsaturated carboxylic acids and monomers copolymerizable therewith. In the case of the copolymers, the ratio of the unsaturated carboxylic acid units are preferably at least 50 mol% with respect to the total structure units. An ethylenically unsaturated polymerizable monomer is preferable as the copolymerizable monomer, and examples thereof include styrene, acrylamide, acrylonitrile, methyl methacrylate, acrylic acid salts, vinyl chloride, allyl chloride, vinyl acetate, and 1,1,6-trimethylhexamethylene dimethacrylate ester. Among those polyalkenoic acids, a homopolymer or copolymer of acrylic acid or maleic acid is preferable. When these polyalkenoic acids have a weight-average molecular weight of less than 5,000, the cured product of the dental cement composition may have reduced strength and poor durability. On the other hand, when it has a weight-average molecular weight exceeding 40,000, it may have high consistency during mixing of the dental cement composition and therefore may have lower operability. Accordingly, a preferable weight-average molecular weight of the polyalkenoic acid is 5,000 to 40,000.

From the viewpoints of curability in the acid-base reaction and the property of sustained-release of fluoride of the composition, the filler (F) to be used is preferably at least one selected from the group consisting of fluoroaluminosilicate glass, calcium fluoroaluminosilicate glass, strontium fluoroaluminosilicate glass, barium fluoroaluminosilicate glass, and strontium calcium fluoroaluminosilicate glass, and more preferably fluoroaluminosilicate glass and/or barium fluoroaluminosilicate glass.

Furthermore, the solvent (E) to be used is not particularly limited. However, from the viewpoint that an acid-base reaction is allowed to proceed smoothly, it is preferable that the solvent (E) contain water (J). When a mixed solvent of water (J) and the organic solvent (K) is used as the solvent (E), the content of water (J) in the mixed solvent is preferably at least 50 mass%, more preferably at least 70 mass%, and further preferably at least 90 mass%. In an embodiment in which particularly progress of the acid-base reaction is considered important, it is particularly preferable that the solvent (E) consist substantially of water (J) alone.

When the polymerizable composition of the present invention is used as a glass ionomer cement, particularly preferably as a resin-reinforced glass ionomer cement, the amounts of the respective components to be added are not particularly limited. When the polymerizable composition contains (d-1) and (d-2), it contains preferably 1 to 99 parts by mass of (d-1), 1 to 90 parts by mass of (d-2), and 0 to 50 parts by mass of (d-3), and more preferably 2 to 95 parts by mass of (d-1), 5 to 85 parts by mass of (d-2), and 0 to 30 parts by mass of (d-3), in 100 parts by mass of the polymerizable monomer (D). When the polymerizable composition contains (d-1) and (d-4), it contains preferably 1 to 99 parts by mass of (d-1), 1 to 90 parts by mass of (d-4), and 0 to 50 parts by mass of (d-3), and more preferably 2 to 95 parts by mass of (d-1), 5 to 98 parts by mass of (d-4), and 0 to 30 parts by mass of (d-3), in 100 parts by mass of the polymerizable monomer (D). Furthermore, when the polymerizable composition contains (d-1), (d-2), and (d-4), it contains preferably 1 to 98 parts by mass of (d-1), 1 to 90 parts by mass of (d-2), 1 to 90 parts by mass of (d-4), and 0 to 50 parts by mass of (d-3), and more preferably 2 to 90 parts by mass of (d-1), 5 to 85 parts by mass of (d-2), 5 to 85 parts by mass of (d-4), and 0 to 30 parts by mass of (d-3), in 100 parts by mass of the polymerizable monomer (D).

Moreover, the content of (F) is preferably 30 to 2000 parts by mass, and more preferably 50 to 1500 parts by mass, with respect to 100 parts by mass of the polymerizable monomer (D). When the content of (F) is less than 30 parts by mass, mechanical strength of the cured product may be insufficient. On the other hand, when the content of (F) exceeds 2000 parts by mass, the polymerizable composition paste has lower fluidity, which makes sufficient mixing difficult, and therefore the acid-base reaction may not proceed smoothly. As a result, the cured product may have reduced strength.

With respect to 100 parts by mass of the polymerizable monomer (D), the content of the solvent (E) is preferably 7 to 500 parts by mass, more preferably 10 to 300 parts by mass, and further preferably 20 to 100 parts by mass. When the solvent (E) is contained in such ranges, the acid-base reaction can proceed smoothly, and the resultant cured product has excellent mechanical strength and excellent adhesive properties to a tooth structure.

With respect to 100 parts by mass of the polymerizable monomer (D), the content of the aforementioned polyalkenoic acid is preferably 1 to 200 parts by mass, more preferably 5 to 100 parts by mass, and further preferably 10 to 50 parts by mass. When the polyalkenoic acid is contained in such ranges, curing through the acid-base reaction proceeds smoothly and decay of the resultant cured product inside an oral cavity by, for example, hydrolysis can be diminished.

As described above, since curing of a glass ionomer cement occurs through progress of an acid-base reaction, from the viewpoint of storage stability, it is preferable that a basic filler (F) and polyalkenoic acid be packed in separate containers and be used after being mixed immediately before use. The preferable types of products to be employed include a so-called powder-liquid type, but from the viewpoint of improving handling ability, the form of so-called two past-type glass ionomer cement containing two types of pastes is more preferable. In the case where the type of product is the two paste type, when the aforementioned two pastes are referred to as a paste A and a paste B, respectively, an embodiment is preferable in which the paste A contains (d-1), (I), (E), (F), and polyalkenoic acid and the paste B contains (d-2) and (F). Furthermore, an embodiment in which the paste A contains (d-1), (I), (E), (F), and polyalkenoic acid and the paste B contains (d-4) and (F) also is used preferably. In addition, an embodiment in which the paste A contains (d-4), (I), (F), and polyalkenoic acid and the paste B contains (d-1). (G), (F), and (E) also is used preferably. In this case, when particularly adhesive properties are considered important, it is preferable that the paste A further contain (d-3), and from the similar viewpoint, it also is preferable that the paste B further contain (d-2). In either of the embodiments, since the paste A contains polyalkenoic acid, it is preferable that at least one selected from the group consisting of fluoroaluminosilicate glass, calcium fluoroaluminosilicate glass, strontium fluoroaluminosilicate glass, barium fluoroaluminosilicate glass, and strontium calcium fluoroaluminosilicate glass be used as the filler (F) contained in the paste B, and it is more preferable that fluoroaluminosilicate glass and/or barium fluoroaluminosilicate glass be used as the filler (F) contained in the paste B. On the other hand, the filler (F) contained in the paste A to be used is preferably one that exhibits no reactivity with polyalkenoic acid, and particularly preferably quartz.

These dental materials can be prepared and used according to a conventional method. These dental materials exhibit high curability and excellent bond strength to a tooth structure (particularly dentin) not only when they are photocured using a halogen lamp-curing unit but also when they are photocured using an LED light-curing unit. In addition, these dental materials hardly have discoloration and adhesive properties deterioration even when stored for a long time, so that they have excellent storage stability.

### Examples

Hereinafter, the present invention is described in further detail using examples but is not limited thereto.

Experiment 1 Evaluation 1 on thickness of unpolymerized portion of polymerizable composition (study on content ratio between bisacylphosphine oxide and α-diketone)

### (1) Preparation of polymerizable composition

Bis(2,4,6-trimethylbenzoyl)phenylphosphine oxide (BAPO) was prepared as the bisacylphosphine oxide (A). Camphorquinone (CQ) was prepared as the α-diketone (B). 4-N,N-dimethylaminobenzoic acid ethyl ester (PDE) was prepared as the tertiary amine (C). 1,2-Bis(3-methacryloyloxy-2-hydroxypropoxy)ethane (#801) was prepared as the polymerizable monomer (D). Polymerizable compositions having the following compositions were produced using these components.

**[Table 1]**

| Bisacylphosphine oxide/α-diketone ratio and thicknesses of unpolymerized portion | | | | | | | |
|---|---|---|---|---|---|---|---|
| | #801 | BAPO | CQ | PDE | BAPO/CQ ratio | Thicknesses of unpolymerized portion (µm) | |
| | | | | | | Halogen | LED |
| Composition 1-1 | 50 | 2.00 | 0 | 0.50 | 1/0 | 4.8 | 14.8 |
| Composition 1-2 | | 2.00 | 0 | 0 | 1/0 | 4.0 | 14.0 |
| Composition 1-3 | | 1.80 | 0.20 | 0.10 | 9/1 | 4.3 | 8.6 |
| Composition 1-4 | | 1.67 | 0.33 | 0.17 | 5/1 | 4.6 | 7.6 |
| Composition 1-5 | | 1.50 | 0.50 | 0.25 | 3/1 | 4.7 | 7.4 |
| Composition 1-6 | | 1.33 | 0.67 | 0.34 | 2/1 | 4.9 | 8.0 |
| Composition 1-7 | | 1.00 | 1.00 | 0.50 | 1/1 | 5.2 | 6.6 |
| Composition 1-8 | | 0.67 | 1.33 | 0.67 | 1/2 | 6.0 | 6.3 |
| Composition 1-9 | | 0.50 | 1.50 | 0.75 | 1/3 | 7.2 | 7.4 |
| Composition 1-10 | | 0.33 | 1.67 | 0.84 | 1/5 | 7.7 | 6.8 |
| Composition 1-11 | | 0.20 | 1.80 | 0.90 | 1/9 | 8.4 | 7.0 |
| Composition 1-12 | | 0 | 2.00 | 1.00 | 0/1 | 14.0 | 7.5 |

| | | | | | | | |
|---|---|---|---|---|---|---|---|
| (The amounts of respective components added each are indicated in the unit of parts by weight.) | | | | | | | |

"Halogen" and "LED" in the table indicate below.
Halogen: Irradiated with light using "JET LITE 3000", a dental visible light-curing unit (manufactured by J. Morita USA)
LED: Irradiated with light using "LED DEMETRON I", a dental visible light-curing unit (manufactured by Kerr Corp.)

### (2) Measurement of thickness of unpolymerized portion

A polyester film was placed on slide glass, and a Teflon (registered trademark) mold with a φ10 mm hole and a thickness of 1 mm was placed thereon. The polymerizable composition produced above was filled thereinto with a plastic syringe and irradiated with light using "JET LITE 3000", a dental visible light-curing unit (manufactured by J. Morita USA), or "LED DEMETRON I", a dental visible light-curing unit (manufactured by Kerr Corp.) for 10 seconds. Then, an uncured portion of the polymerizable composition was wiped off with a JK wiper, and the thickness of the uncured portion was calculated from the weight reduced. Table 1 shows the measurement results thereof.

As shown in Table 1, as for the polymerizable compositions (1-1 and 1-2) containing no α-diketone (B), the thickness of the unpolymerized portion was large and the curability was insufficient when they were irradiated with light using the LED light-curing unit. As for the polymerizable composition (1-12) containing no bisacylphosphine oxide (A), the curability was lowered when it was irradiated with light using the halogen lamp-curing unit. In contrast, as for the polymerizable compositions in which the ratio (A):(B) between the bisacylphosphine oxide (A) and the α-diketone (B) was in the range of 1:9 to 9:1, the thickness of the unpolymerized portion was small and excellent curability was obtained both when they were irradiated with light using the halogen lamp-curing unit and when they were irradiated with light using the LED light-curing unit.

Experiment 2 Evaluation 2 on thickness of unpolymerized portion of polymerizable composition (study on content ratio between α-diketone and tertiary amine)

### (1) Preparation of polymerizable composition

BAPO was prepared as the bisacylphosphine oxide (A). CQ was prepared as the α-diketone (B). PDE was prepared as the tertiary amine (C). #801 was prepared as the polymerizable monomer (D). Polymerizable compositions having the following compositions were produced using these components.

**[Table 2]**

| α-Diketone/tertiary amine ratio and thicknesses of unpolymerized portion | | | | | | | |
|---|---|---|---|---|---|---|---|
| | #801 | BAPO | CQ | PDE | CQ/PDE ratio | Thicknesses of unpolymerized portion (µm) | |
| | | | | | | Halogen | LED |
| Composition 2-1 | 5 | 0.03 | 0.07 | 0 | 1/0 | 10.3 | 21.3 |
| Composition 2-1 | | | | 0.014 | 5/1 | 9.2 | 10.2 |
| Composition 2-2 | | | | 0.023 | 3/1 | 8.5 | 7.0 |
| Composition 2-3 | | | | 0.035 | 2/1 | 7.2 | 7.5 |
| Composition 2-4 | | | | 0.07 | 1/1 | 9.5 | 10.6 |
| Composition 2-5 | | | | 0.14 | 1/2 | 11.0 | 9.8 |
| Composition 2-6 | | | | 0.21 | 1/3 | 11.7 | 10.8 |
| Composition 2-7 | | | | 0.35 | 1/5 | 11.9 | 12.7 |
| Composition 2-8 | | | | 0.7 | 1/10 | 12.4 | 12.6 |

| | | | | | | | |
|---|---|---|---|---|---|---|---|
| (The amounts of respective components added each are indicated in the unit of parts by weight.) | | | | | | | |

### (2) Measurement of thickness of unpolymerized portion

The thicknesses of the unpolymerized portions were measured in the same manner as in Experiment 1. Table 2 shows the measurement results thereof

As shown in Table 2, as for the polymerizable composition (2-1) containing no tertiary amine (C), the thickness of the unpolymerized portion was large and the curability was insufficient when it was irradiated with light using the LED light-curing unit. In contrast, as for the polymerizable compositions containing the α-diketone (B) and the tertiary amine at a preferable ratio, the thickness of the unpolymerized portion was small and excellent curability was obtained not only when they were irradiated with light using the halogen lamp-curing unit but also when they were irradiated with light using the LED light-curing unit.

### Example 1 Application of the polymerizable composition of the present invention to one-step bonding system (one-component bonding material)

### (1) Preparation of one-component bonding material

One-component bonding materials containing the polymerizable composition of the present invention were produced as examples. Table 3 shows the compositions thereof. Additionally, as comparative examples, there were produced: one-component bonding materials, each using a polymerizable composition obtained by omitting an essential component from the polymerizable composition of the present invention; one-component bonding materials, each using a polymerizable composition in which the weight ratio between the bisacylphosphine oxide (A) and the α-diketone was out of the range of the present invention; one-component bonding materials, each using a polymerizable composition in which the content of the tertiary amine (C) was out of the range of the present invention and a one-component bonding material using a polymerizable composition that further contains coumarin, a common photopolymerization initiator. Table 4 shows the compositions of the comparative examples.

**[Table 3]**

| Compositions of one-component bonding materials and bonding evaluation results of examples | | | | | | | | | | | | | | |
|---|---|---|---|---|---|---|---|---|---|---|---|---|---|---|
| Component | | Example | | | | | | | | | | | | |
| | | 1 | 2 | 3 | 4 | 5 | 6 | 7 | 8 | 9 | 10 | 11 | 12 | 13 |
| Polymerizable monomer (D) | MPD | 10 | 10 | 10 | 10 | 10 | 10 | 10 | 10 | 10 | 10 | 10 | 10 | 10 |
| | Bis-GMA | 20 | 20 | 20 | 20 | 20 | 20 | 20 | 20 | 20 | 20 | 20 | 20 | 20 |
| | HEMA | 20 | 20 | 20 | 20 | 20 | 20 | 20 | 20 | 20 | 20 | 20 | 20 | 20 |
| | UDMA | | | | | | | | | | 5 | | | |
| | #801 | | | | | | | | | 5 | | | | |
| | EDMA | 20 | 20 | 20 | 20 | 20 | 20 | 20 | 20 | 15 | 15 | 20 | 20 | 20 |
| Solvent | Distilled water | 15 | 15 | 15 | 15 | 15 | 15 | 15 | 15 | 15 | 15 | 15 | 15 | 15 |
| | Ethanol | 15 | 15 | 15 | 15 | 15 | 15 | 15 | 15 | 15 | 15 | 15 | 15 | 15 |
| Bisacylphosphine oxide (A) | BAPQ | 0.5 | 1 | 1.5 | 2 | 1.5 | 0.5 | 2 | 2 | 2 | 2 | 2 | 0.5 | 1.5 |
| For comparison with (A) | TMDPO | | | | | | | | | | | | | |
| α-diketone (B) | CQ | 0.5 | 1 | 1.5 | 2 | 0.5 | 1.5 | 2 | 2 | 2 | 2 | 2 | 0.5 | 1.5 |
| Tertiary amine (C) | DBE | | | | | | | 1 | | | | | | |
| | PDE | 0.2 | 0.5 | 0.8 | 1 | 0.3 | 0.3 | | 1 | 1 | 1 | 1 | 0.2 | |
| | Triethanolamine | 0.5 | 0.5 | 0.5 | 0.5 | 0.5 | 0.5 | 0.5 | | 0.5 | 0.5 | 0.5 | 0.2 | 1.5 |
| | DEPT | | | | | | | | 1 | | | | | |
| Polymerization inhibitor | BHT | 0.1 | 0.1 | 0.1 | 0.1 | 0.1. | 0.1 | 0.1 | 0.1 | 0.1 | 0.1 | 0.1 | 0.1 | 0.1 |
| Additive | Sodium fluoride | | | | | | | | | | | 0.3 | | |
| Filler | Inorganic filler 1 | 5 | 5 | 5 | 5 | 5 | 5 | 5 | 5 | 5 | 5 | 5 | 5 | 5 |
| Bond strength to dentin (MPa) | | 17.1 | 19.4 | 21.4 | 20.1 | 19.9 | 20.2 | 20.7 | 19.2 | 18.8 | 17.8 | 20.0 | 17.3 | 16.8 |
| Storage stability evaluation (stored at 50°C for four weeks) | Bond strength (MPa) | 14.4 | | 17.6 | | 15.9 | | | | | | | 13.9 | 13.7 |
| | Degree of discoloration of the composition | Good | | Good | | Good | | | | | | | Good | Good |
| (A)/(B) | | 1/1 | 1/1 | 1/1 | 1/1 | 3/1 | 1/3 | 1/1 | 1/1 | 1/1 | 1/1 | 1/1 | 1/1 | 1/1 |
| (D)/(C) | | 100/1 | 100/1.4 | 100/1.9 | 100/2.1 | 100/1.1 | 100/1.1 | 100/2.1 | 100/2.9 | 100/2.1 | 100/2.1 | 100/2.1 | 100/0.6 | 100/3.6 |
| (B)/(C) | | 1/1.4 | 1/1 | 1.2/1 | 1.3/1 | 1/1.6 | 1.9/1 | 1.3/1 | 1/1 | 1.3/1 | 1.3/1 | 1.3/1 | 1.3/1 | 1/1.7 |
| Total amount of (A) + (B)with respect to 100 parts by weight of polymerizable monomer | | 1.4 | 2.9 | 4.3 | 5.7 | 2.9 | 2.9 | 5.7 | 5.7 | 5.7 | 5.7 | 5.7 | 1.4 | 4.3 |

| | | | | | | | | | | | | | | |
|---|---|---|---|---|---|---|---|---|---|---|---|---|---|---|
| (The amounts of respective components added each are indicated in the unit of parts by weight.) | | | | | | | | | | | | | | |

**[Table 4]**

| Compositions of one-component bonding material and bonding evaluation results of comparative examples | | | | | | | | | | | |
|---|---|---|---|---|---|---|---|---|---|---|---|
| Component | | Comparative Example | | | | | | | | | |
| | | 1 | 2 | 3 | 4 | 5 | 6 | 7 | 8 | 9 | 10 |
| Polymerizable monomer (D) | MDP | 10 | 10 | 10 | 10 | 10 | 10 | 10 | 10 | 10 | 10 |
| | Bis-CMA | 20 | 20 | 20 | 20 | 20 | 20 | 20 | 20 | 20 | 20 |
| | HEMA | 20 | 20 | 20 | 20 | 20 | 20 | 20 | 20 | 20 | 20 |
| | UDMA | | | | | | | | | | |
| | #801 | | | | | | | | | | |
| | EDMA | 20 | 20 | 20 | 20 | 20 | 20 | 20 | 20 | 20 | 20 |
| Solvent | Distilled water | 15 | 15 | 15 | 15 | 15 | 15 | 15 | 15 | 15 | 15 |
| | Ethanol | 15 | 15 | 15 | 15 | 15 | 15 | 15 | 15 | 15 | 15 |
| Bisacylphosphine oxide (A) | BAPO | 0.2 | 2 | | | | 2 | 2 | 0.5 | 1.5 | 0.5 |
| For comparison with (A) | TMDPO | | | 2 | 2 | | | | | | |
| α-diketone (B) | CQ | 2 | 0.2 | 2 | | 2 | | 2 | 0.5 | 1.5 | 0.5 |
| Coumarin | | | | | | | | | | | 0.5 |
| Tertiary amine (C) | DBE | | | | | | | | | | |
| | PDE | 1. | 0.1 | 1 | 1 | 1 | 1 | | 0.2 | 4.5 | 0.2 |
| | Triethanolamine | 0.5 | 0.5 | 0.5 | 0.5 | 0.5 | 0.6 | | 0. 1. | 3 | 0.5 |
| | DEPT | | | | | | | | | | |
| Polymerization inhibitor | BHT | 0.1 | 0.1 | 0.1 | 0.1 | 0.1 | 0.1 | 0.1 | 0.1 | 0.1 | 0.1 |
| Additive | Sodium fluoride | | | | | | | | | | |
| Filler | Inorganic filler 1 | 5 | 5 | 5 | 5 | 5 | 5 | 5 | 5 | 5 | 5 |
| Bond strength to dentin (MPa) | | 14.9 | 12.5 | 14.6 | Not cured | 14.7 | 11.9 | 12.1 | 17.3 | 12.7 | 5 17.0 |
| Storage stability evaluation (stored at 50°C for four weeks) | Bond strength (MPa) | | 10.1 | | | | | | 10.0 | 10.4 | 14.2 |
| | Degree of discoloration of the composition | | Good | | | | | | Good | Good | Not good |
| (A)/(B) | | 1/10 | 10/1 | 0/1 | 0/0 | 0/1 | 1/0 | 1/1 | 1/1 | 1/1 | 1/1 |
| (D)/(C) | | 100/2.1 | 100/0.9 | 100/2.1 | 100/2.1 | 100/2.1 | 100/2.1 | 100/0 | 100/0.4 | 10011 | 100/1 |
| (B)/(C) | | 1.3/11 | 1/3 | 1.3/1 | 0/1 | 1.3/1 | 0/1 | 1/0 | 1.7/1 | 1/5 | 1/1.4 |
| Total amount of (A) + (B) with respect to 100 parts by weight of polymerizablr monomer | | 3.1 | 3.1 | 2.9 | 0 | 2.9 | 2.9 | 5.7 | 1.4 | 4.3 | 1.4 |

| | | | | | | | | | | | |
|---|---|---|---|---|---|---|---|---|---|---|---|
| (The amounts or respective components added each are indicated in the unit of parts by weight.) | | | | | | | | | | | |

The abbreviations in the tables have the following meanings, except for those mentioned above.
MDP: 10-methacryloyloxydecyl dihydrogenphosphate
Bis-GMA: Bisphenol A diglycidyl methacrylate
HEMA: 2-hydroxyethylmethacrylate
UDMA: N,N'-(2,2,4-trimethylhexamethylene)bis[2-(aminocarboxy)ethan-1-ol] dimethacrylate
EDMA: erythritol dimethacrylate [1.4-bis(methacryloyloxy)-2,3-butanediol]
TMDPO: 2,4,6-trimethylbenzoyldiphenylphosphine oxide
DBE: N,N-dimethylaminobenzoic acid n-butoxyethyl ester
DEPT: N,N'di(2'hydroxyethyl)-p-toluidine
BHT: dibutylhydroxytoluene(2,6-di-tert-butyl-p-cresol)
Inorganic filler 1: "R972" manufactured by Japan Aerosil Inc.

### (2) Method of evaluating bonding to bovine teeth dentin

The labial surface of a bovine mandibular incisor was ground with #80 silicon carbide paper (manufactured by Nihon Kenshi Co., Ltd.) under running water, and thereby a sample with an exposed flat surface of dentin was obtained. The sample thus obtained further was ground with #1000 silicon carbide paper (manufactured by Nihon Kenshi Co., Ltd.) under running water. After completion of grinding, water on the surface was air-blown to be dried. An adhesive tape with a thickness of about 150 µm having a circular hole whose diameter was 3 mm was attached to the smooth surface that had been dried and thereby the adhesive area was defined.

Each one-component bonding material produced above was applied into the above-mentioned circular hole using a brush and was then allowed to stand for 10 seconds. Thereafter, the surface thereof was air-blown and thereby the one-component bonding material thus applied was dried until it lost fluidity. Subsequently, it was irradiated with light using "LED DEMETRON I", a dental visible light-curing unit (manufactured by Kerr Corp.), for 10 seconds. Thus, the one-component bonding material applied was cured.

A composite resin for dental filling (trade name "Clearfil AP-X" (registered trademark), produced by Kuraray Medical Inc.) was applied on a surface of the cured body of the one-component bonding material thus formed, and covered with a mold releasing film (made of polyester). Next, slide glass was placed on the mold release film to press it, and thereby the surface of the applied composite resin was smoothed. Subsequently, the composite resin was irradiated with light for 20 seconds using the light-curing unit "LED DEMETRON I" through the mold release film. Thus, the composite resin was cured.

One end face (circular section) of a stainless-steel cylindrical rod (with a diameter of 7 mm and a length of 2.5 cm) was bonded to the surface of the resultant cured product of the composite resin for dental filling using a commercially available dental resin cement (manufactured by Kuraray Medical Inc., "PANAVIA21"). After bonding, this sample was allowed to stand still at room temperature for 30 minutes and was then immersed in distilled water. Ten bonding test samples were produced in total, and allowed to stand still for 24 hours inside a thermostat whose temperature was maintained at 37°C. The obtained samples were measured for bond strength.

### (3) Bonding evaluation test (evaluation of bond strength)

The tensile bond strengths of the above-mentioned bonding test samples were measured with a universal testing machine (manufactured by Shimadzu Corporation), with the crosshead speed being set at 2 mm/min, and the average value thereof was taken as tensile bond strength.

### (4) Storage stability test

The one-component bonding materials produced above were kept in a thermostat at 50°C for four weeks and returned to room temperature, and then measured for bond strength to the dentin to be evaluated. The compositions were also evaluated visually for degree of discoloration. A sample with no discoloration was judged as "good", and one with discoloration was judged as "not good".

Tables 3 and 4 show the results of these evaluations. As shown in Tables 3 and 4, when the polymerizable compositions of examples were photocured using the LED light-curing unit, the cured products thereof exhibited high bond strength to a tooth structure. Also, the polymerizable compositions of examples had high storage stability. In contrast, as for the polymerizable compositions of the comparative examples, even when the composition contained the photopolymerization initiator composition consisting of camphorquinone and PDE, which showed small thicknesses of the unpolymerized portions in Table 1, the composition was inferior in terms of bond strength. Moreover, even when the composition contained the photopolymerization initiator composition containing a small amount of a tertiary amine, which showed small thicknesses of the unpolymerized portions in Tables 1 and 2, the composition had lower bond strength after storage and was inferior in terms of storage stability. Furthermore, even when the composition contained the photopolymerization initiator composition containing a large amount of a tertiary amine, the composition had low bond strength after storage and was inferior in terms of storage stability also. In addition, when the composition contained the photopolymerization initiator composition further contained coumarin, the compositions had discoloration during storage and was inferior in terms of storage stability.

### Industrial Applicability

The polymerizable composition of the present invention can be used in applications that require photocurability. Particularly, the polymerizable composition of the present invention is suitable for dental applications, and as a dental material that is photocured using an LED light-curing unit, and it exhibits high curability, excellent adhesive properties, and high storage stability.

## Claims

1. A polymerizable composition comprising: a photopolymerization initiator composition containing a photopolymerization initiator component consisting of only a bisacylphosphine oxide (A) and α-diketone (B), and tertiary amine (C); and a polymerizable monomer (D), wherein
a weight ratio (A):(B) between the bisacylphosphine oxide (A) and the α-diketone (B) is 1:9 to 9:1, and
a weight ratio (D):(C) between the polymerizable monomer (D) and the tertiary amine (C) is 100:0.5 to 100:10.

2. The polymerizable composition according to claim 1, wherein the bisacylphosphine oxide (A) is a compound represented by formula (1) below: 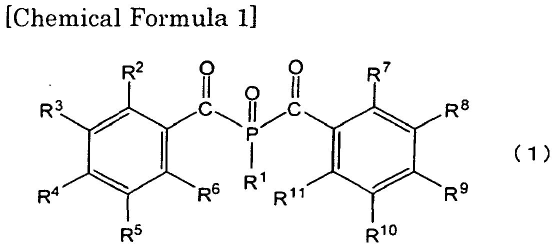 where R¹ denotes an alkyl group, alkenyl group, alkynyl group, aryl group, alkoxy group, acyl group, or acyloxy group, and R² to R¹¹ each denote independently a hydrogen atom, halogen atom, alkyl group, alkenyl group, alkynyl group, aryl group, alkoxy group, acyl group, or acyloxy group.

3. The polymerizable composition according to claim 1, wherein a weight ratio (B):(C) between the α-diketone (B) and the tertiary amine (C) is 1:10 to 5:1.

4. The polymerizable composition according to claim 1, wherein a component involved in photopolymerization consists essentially of the bisacylphosphine oxide (A), the α-diketone (B), and the tertiary amine (C).

5. The polymerizable composition according to claim 1, wherein the polymerizable monomer (D) is at least one polymerizable monomer selected from the group consisting of: a polymerizable monomer (d-1) having an unconjugated carbon chain with at least four carbon atoms bonded continuously, at least two polymerizable groups, and at least two hydroxyl groups; a polymerizable monomer (d-2) having one polymerizable group and at least one hydroxyl group; a polymerizable monomer (d-3) having an acidic group; and a crosslinkable polymerizable monomer (d-4) other than the polymerizable monomer (d-1).

6. The polymerizable composition according to claim 5, wherein the polymerizable monomer (d-1) is a compound represented by formula (2) below: 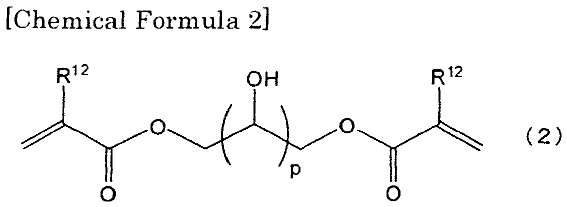 where R¹² denotes a hydrogen atom or an aliphatic hydrocarbon group having 1 to 10 carbon atoms, and p denotes an integer of 2 or more.

7. The polymerizable composition according to claim 1, wherein a total content of the bisacylphosphine oxide (A) and the α-diketone (B) is 0.1 to 20 parts by weight with respect to 100 parts by weight of the polymerizable monomer (D).

8. The polymerizable composition according to claim 1, being for a dental application.

9. A dental bonding material using the polymerizable composition according to claim 1.

## Patentansprüche

1. Polymerisierbare Zusammensetzung, umfassend: eine Photopolymerisationsinitiatorzusammensetzung, die eine Photopolymerisationsinitiatorkomponente, die nur aus einem Bisacylphosphinoxid (A) und α-Diketon (B) besteht, und ein tertiäres Amin enthält, und ein polymerisierbares Monomer (D), wobei
ein Gewichtsverhältnis (A):(B) zwischen dem Bisacylphosphinoxid (A) und dem α-Diketon (B) 1:9 bis 9:1 beträgt, und
ein Gewichtsverhältnis (D):(C) zwischen dem polymerisierbaren Monomer (D) und dem tertiären Amin (C) 100:0,5 bis 100:10 beträgt.

2. Polymerisierbare Zusammensetzung nach Anspruch 1, wobei das Bisacylphosphinoxid (A) eine Verbindung ist, die durch die unten gezeigte Formel (1) dargestellt wird: 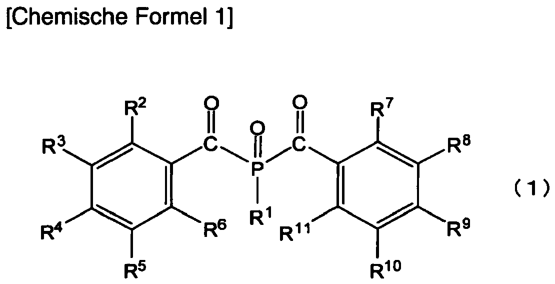 wobei R¹ eine Alkylgruppe, eine Alkenylgruppe, eine Alkynylgruppe, eine Arylgruppe, eine Alkoxygruppe, eine Acylgruppe oder eine Acyloxygruppe bezeichnet und R² bis R¹¹ jeweils unabhängig ein Wasserstoffatom, ein Halogenatom, eine Alkylgruppe, eine Alkenylgruppe, eine Alkynylgruppe, eine Arylgruppe, eine Alkoxygruppe, eine Acylgruppe oder eine Acyloxygruppe bezeichnen.

3. Polymerisierbare Zusammensetzung nach Anspruch 1, wobei ein Gewichtsverhältnis (B):(C) zwischen dem α-Diketon (B) und dem tertiären Amin (C) 1:10 bis 5:1 beträgt.

4. Polymerisierbare Zusammensetzung nach Anspruch 1, wobei eine in der Photopolymerisation involvierte Komponente im wesentlichen aus dem Bisacylphosphinoxid (A), dem α-Diketon (B) und dem tertiären Amin (C) besteht.

5. Polymerisierbare Zusammensetzung nach Anspruch 1, wobei das polymerisierbare Monomer (D) wenigstens ein polymerisierbares Monomer ist, das aus der aus einem polymerisierbaren Monomer (d-1), das eine unkonjugierte Kohlenstoffkette aus wenigstens vier kontinuierlich gebundenen Kohlenstoffatomen, wenigstens zwei polymerisierbare Gruppen und wenigstens zwei Hydroxylgruppen aufweist, einem polymerisierbaren Monomer (d-2), das eine polymerisierbare Gruppe und wenigstens eine Hydroxylgruppe aufweist, einem polymerisierbaren Monomer (d-3), das eine saure Gruppe aufweist, und einem quervernetzbaren polymerisierbaren Monomer (d-4), das anders als das polymerisierbare Monomer (d-1) ist, bestehenden Gruppe ausgewählt ist.

6. Polymerisierbare Zusammensetzung nach Anspruch 5, wobei das polymerisierbare Monomer (d-1) eine Verbindung ist, die durch nachfolgende Formel (2) dargestellt wird: 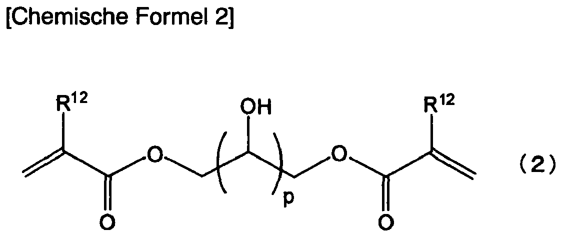 wobei R¹² ein Wasserstoffatom oder eine aliphatische Kohlenwasserstoffgruppe mit 1 bis 10 Kohlenstoffatomen bezeichnet und p eine ganze Zahl von 2 oder größer bezeichnet.

7. Polymerisierbare Zusammensetzung nach Anspruch 1, wobei ein Gesamtgehalt des Bisacylphosphinoxids (A) und des α-Diketons (B) 0,1 bis 20 Gewichtsanteile bezüglich 100 Gewichtsanteilen des polymerisierbaren Monomers (D) beträgt.

8. Polymerisierbare Zusammensetzung nach Anspruch 1, für dentale Anwendung.

9. Dentales Klebematerial, das die polymerisierbare Zusammensetzung nach Anspruch 1 verwendet.

## Revendications

1. Composition polymérisable comprenant : une composition d'initiateur de photopolymérisation contenant un constituant d'initiateur de photopolymérisation constitué uniquement d'un oxyde de bisacylphosphine (A) et d'α-dicétone (B), et d'amine tertiaire (C) ; et un monomère polymérisable (D), dans laquelle
un rapport massique (A):(B) entre l'oxyde de bisacylphosphine (A) et l'α-dicétone (B) est de 1:9 à 9:1, et
un rapport massique (D):(C) entre le monomère polymérisable (D) et l'amine tertiaire (C) est de 100:0,5 à 100:10.

2. Composition polymérisable selon la revendication 1, dans laquelle l'oxyde de bisacylphosphine (A) est un composé représenté par la formule (1) ci-dessous: 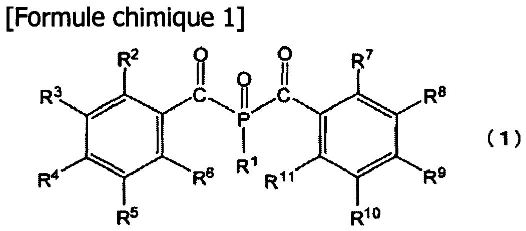 où R¹ indique un groupe alkyle, un groupe alcényle, un groupe alcynyle, un groupe aryle, un groupe alcoxy, un groupe acyle, ou un groupe acyloxy, et R² à R¹¹ indiquent chacun indépendamment un atome d'hydrogène, un atome d'halogène, un groupe alkyle, un groupe alcényle, un groupe alcynyle, un groupe aryle, un groupe alcoxy, un groupe acyle, ou un groupe acyloxy.

3. Composition polymérisable selon la revendication 1, dans laquelle un rapport massique (B):(C) entre l'α-dicétone (B) et l'amine tertiaire (C) est de 1:10 à 5:1.

4. Composition polymérisable selon la revendication 1, dans laquelle un constituant impliqué dans la photopolymérisation est essentiellement constitué de l'oxyde de bisacylphosphine (A), de l'α-dicétone (B), et de l'amine tertiaire (C).

5. Composition polymérisable selon la revendication 1, dans laquelle le monomère polymérisable (D) est au moins un monomère polymérisable choisi dans le groupe constitué de : un monomère polymérisable (d-1) présentant une chaîne carbonée non conjuguée avec au moins quatre atomes de carbone liés en continu, au moins deux groupes polymérisables, et au moins deux groupes hydroxyle ; un monomère polymérisable (d-2) présentant un groupe polymérisable et au moins un groupe hydroxyle ; un monomère polymérisable (d-3) présentant un groupe acide ; et un monomère polymérisable réticulable (d-4) différent du monomère polymérisable (d-1).

6. Composition polymérisable selon la revendication 5, dans laquelle le monomère polymérisable (d-1) est un composé représenté par la formule (2) ci-dessous : 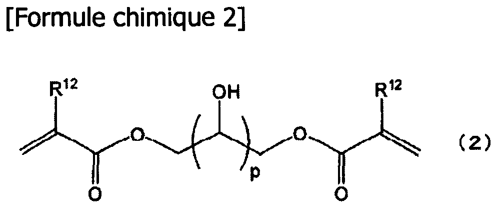 où R¹² indique un atome d'hydrogène ou un groupe hydrocarboné aliphatique ayant de 1 à 10 atomes de carbone, et p indique un nombre entier de 2 ou plus.

7. Composition polymérisable selon la revendication 1, dans laquelle une teneur totale de l'oxyde de bisacylphosphine (A) et de l'α-dicétone (B) est de 0,1 à 20 parties en masse rapporté à 100 parties en masse du monomère polymérisable (D).

8. Composition polymérisable selon la revendication 1, étant destinée à une application dentaire.

9. Matériau de liaison dentaire utilisant la composition polymérisable selon la revendication 1.
